# DEMANDE DE BREVET EUROPEEN

(11) **EP 1 201 266 A1**
(43) Date de publication de la demande: **02.05.2002**
(21) Numéro de dépôt: 00203742.2
(22) Date de dépôt: 26.10.2000
(51) Int. Cl.: A61N 1/36, G06F 19/00

(54) **Procédé de programmation de données de stimulation électrique pour un appareil de stimulation électrique, et installation pour sa mise en oeuvre**

(71) Demandeur: Compex SA, 1024 Ecublens (CH)
(72) Inventeur: Popovic, Milos R., Toronto, Ontario, M5P 3G9 (CA); Keller, Thierry, 8006 Zürich (CH); Pappas Ion P., 8032 Zürich (CH)
(74) Mandataire: Ravenel, Thierry Gérard Louis

(57) **Abrégé**

Le procédé de programmation de données de stimulation électrique pour un appareil de stimulation électrique neuromusculaire ou fonctionnelle (2) est réalisé dans une installation, qui comprend une station d'ordinateur (1) à programme de gestion de données de stimulation électrique et à interface utilisateur graphique (4), au moins un dispositif de lecture et d'écriture en communication avec la station d'ordinateur (1), et au moins un support amovible de mémorisation, tel qu'une carte à puce (8), placé dans le dispositif pour mettre en mémoire des données personnelles de stimulation électrique. Dans une fenêtre principale de l'interface graphique, au moins une séquence de données de stimulation est composée avec des blocs unitaires de fonctions de stimulation représentés chacun par une icône spécifique de leur fonction et tirés d'une fenêtre de bibliothèque de blocs unitaires. La séquence composée est ensuite traduite en une séquence codée pour être transmise par un câble électrique (5) et enregistrée sur le support de mémorisation (8).

L'appareil de stimulation électrique (2) peut être une neuroprothèse dans laquelle les séquences codées de la carte à puce sont traduites inversement par un programme de gestion complémentaire pour générer notamment des trains d'impulsions à fournir à des électrodes de stimulation.

## Description

L'invention concerne un procédé de programmation de données de stimulation électrique pour un appareil de stimulation électrique neuromusculaire ou fonctionnelle dans une installation qui comprend une station d'ordinateur à programme de gestion de données de stimulation électrique et à interface utilisateur graphique, au moins un dispositif de lecture et d'écriture en communication avec la station d'ordinateur, et au moins un support de mémorisation placé dans le dispositif pour mettre en mémoire des données personnelles de stimulation électrique.

L'invention concerne également une installation de programmation de données de stimulation électrique notamment pour la mise en oeuvre du procédé. L'installation comprend une station d'ordinateur ayant une unité centrale de traitement à programme de gestion de données de stimulation électrique et une interface utilisateur graphique, au moins un dispositif de lecture et d'écriture en communication avec la station d'ordinateur, et au moins un support de mémorisation placé dans le dispositif pour mettre en mémoire des données personnelles de stimulation électrique.

De manière à faciliter l'établissement de séquences d'impulsions électriques de stimulation à imposer à un utilisateur par l'intermédiaire d'un appareil de stimulation électrique neuromusculaire ou fonctionnelle, une interface graphique d'une station d'ordinateur est prévue pour un programmeur. Ce dernier peut ainsi visualiser les séquences d'impulsions électriques qu'il introduit et qu'il enregistre pour un utilisateur spécifique.

Il est à noter que les séquences de données de stimulation composées dans l'installation de programmation sont complexes pour opérer un mouvement défini d'un membre du corps humain à l'aide d'un appareil de stimulation électrique. L'utilisation d'une telle installation est de ce fait adaptée avantageusement à un usage médical ou thérapeutique pour des personnes handicapées ou accidentées.

Les données de stimulation électrique employées dans la présente invention concernent aussi bien des données textuelles à afficher sur un écran de la station d'ordinateur ou sur un écran d'un appareil de stimulation, des données sonores d'avertissement, des données relatives à des capteurs ou à des actionneurs en tant que dispositif d'interaction de l'utilisateur, des données de sélection de canaux de stimulation, des données de boucles de répétition de séquences de stimulation, des données de branchement dans les séquences de stimulation, des données de rampes d'amplitude ou de largeur d'impulsions et des données d'amplitude, de fréquence, de largeur des impulsions électriques.

Des appareils de stimulation électrique neuromusculaire ou fonctionnelle peuvent servir dans divers buts. Ils sont utilisés en outre pour le renforcement de la musculature à travers des programmes d'entraînements en puissance ou d'endurance, pour maintenir ou accroître la marge de mouvement, pour faciliter ou rééduquer les fonctions motrices volontaires, ou pour d'autres buts.

Dans le domaine médical, l'utilisation de tels appareils de stimulation électrique pour l'aide aux personnes handicapées ou accidentées s'est développée ces dernières années. Cela a ainsi permis à ces personnes de retrouver certaines facultés physiques perdues ou manquantes. Il a été proposé notamment de permettre à ces personnes de pouvoir commander l'action de leurs jambes, de leurs bras ou de leurs mains paralysés ou accidentés. Pour ce faire, des séquences d'impulsions électriques sont fournies à des électrodes posées sur des points moteurs des muscles à stimuler électriquement. Cette aide procurée par l'appareil de stimulation permet aux personnes handicapées ou accidentées de devenir autant que possible indépendante dans leurs mouvements sans nécessiter l'assistance d'autres personnes.

L'appareil utilisé comme neuroprothèse dans ce domaine médical est défini comme un appareil de stimulation électrique fonctionnelle. Dans ce cas, la génération des impulsions électriques par ledit appareil pour la gestion des mouvements d'un membre du corps humain est planifiée pour une longue période d'activité. Le lecteur peut se référer pour plus de détails concernant la stimulation électrique neuromusculaire, dont fait partie la stimulation électrique fonctionnelle, au guide pratique 3^{e} édition intitulé "NeuroMuscular Electrical Stimulation" de L. L. Baker, D. R. McNeal, L. A. Benton, B. R. Bowman et R. L. Waters publié par Los Amigos Research & Education Institute, Inc. en 1993.

L'agencement des séquences de stimulation de tous les canaux de stimulation d'une neuroprothèse utilisée pour opérer un mouvement d'une main ou de jambes par exemple nécessite une programmation complexe de toutes les données de stimulation électrique. C'est pourquoi, il est proposé d'utiliser une interface graphique pour visualiser les données de stimulation électrique qu'un programmeur doit composer.

Il est utile de rappeler à l'usage de l'électrostimulation médicale qu'un muscle paralysé est un muscle qui présente une déficience ou un manque de fonction motrice, le plus souvent dû à une lésion nerveuse périphérique ou centrale. Si la lésion nerveuse se trouve principalement au niveau du système nerveux central, le système nerveux périphérique reste intact. Cela permet au muscle paralysé, mais pas énervé, d'être stimulé comme un muscle en santé par les moyens d'une stimulation électrique neuromusculaire ou fonctionnelle. Si un muscle est normalement innervé, il est stimulé par excitation de son nerf moteur par exemple au moyen d'impulsions rectangulaires de courant biphasiques de courtes durées de largeur entre 15 à 350 µs. Si par contre les muscles sont énervés, les impulsions rectangulaires doivent être plus longues en durée, par exemple entre 50 à 200 ms. Ces impulsions de longues durées ne doivent en principe pas être biphasiques.

Le but essentiel de l'électro-stimulation, notamment pour une utilisation dans ce domaine médical, est de pouvoir créer des potentiels d'action dans des cellules stimulables. Pour obtenir une excitation, ce n'est pas la forme de l'impulsion qui compte, mais plutôt la quantité de charges électriques. Cette règle reste valable si la durée totale des impulsions électriques reste la même.

Le maintien de la marge de mouvement et l'empêchement de déséquilibre musculaire et de restriction des fibres de contraction d'articulation est un problème clinique constant dans le traitement de patients paralysés par exemple.

Principalement dans ce domaine médical ou également pour un entraînement musculaire, une installation comprenant une station de programmation reliée à un appareil de stimulation électrique neuromusculaire a été décrite notamment dans le brevet EP 0 197 889 de la Demanderesse. L'installation comprend un programmeur, dans lequel un thérapeute par exemple programme initialement des séquences de stimulation qu'il mémorise sur une carte à puce introduite dans un dispositif de lecture et d'écriture du programmeur. Ensuite de quoi, la carte à puce est introduite dans un autre dispositif de lecture et d'écriture de l'appareil de stimulation pour lui permettre de lire les données de stimulation enregistrées sur la carte à puce et de fournir ainsi les trains d'impulsions électriques aux électrodes.

Lors de la mise en fonction des périodes de stimulation de l'appareil sur un utilisateur, une liaison en ligne avec le programmeur peut être maintenue. De cette façon, des corrections des paramètres de stimulation peuvent être enregistrées sur la carte à puce et également dans une mémoire du programmeur. Les corrections sont fournies manuellement ou sur la base de mesures de capteurs des réactions musculaires en temps réel.

Un inconvénient de cette installation consiste en ce que la première opération de chargement de données de stimulation sur la carte à puce, qui est personnalisée au patient à traiter, doit être réalisée sur le programmeur. Ceci nécessite continuellement l'utilisation du programmeur et de l'appareil de stimulation pour adapter tous les paramètres et les séquences de stimulation spécifiques à un patient.

Un autre inconvénient de cette installation est que la programmation des séquences de stimulation à enregistrer sur la carte à puce exige une bonne connaissance des signaux électriques à appliquer au niveau des points moteurs des muscles à stimuler. Des moyens de sécurité sont pourtant prévus pour éviter des programmations de paramètres erronées au-delà de limites préalablement déterminées dans le programmeur et dans l'appareil de stimulation portable.

Pour une utilisation plus spécifique essentiellement dans le domaine médical, la demande de brevet WO 98/55177 décrit un système de stimulation électrique fonctionnelle dans le but de permettre le mouvement de membres du corps paralysés ou accidentés grâce à une neuroprothèse. Le système comprend une station d'ordinateur dans laquelle des données de stimulation électrique sont introduites et enregistrées, un appareil de stimulation portable recevant les données de stimulation établies dans la station d'ordinateur, et une mémoire du système, tel qu'un support d'enregistrement.

La mémoire du système contient un fichier de données qui, sous commande d'un programme du système, produit au moins un motif de stimulation électrique pour les muscles à stimuler. Une table de la mémoire enregistre des informations de génération de commandes et de motifs de stimulation, et séparément des données de caractérisation pour chaque électrode. Des motifs de mouvement requis des muscles à stimuler sont élaborés et enregistrés dans le système pour définir au moins trois mouvements désirés.

Même si la station d'ordinateur permet d'élaborer relativement facilement des profils ou des séquences de stimulation, un enregistrement des données doit être réalisé aussi bien dans une mémoire de la station que dans une mémoire de l'appareil de stimulation. La visualisation de toutes les données de stimulation et caractéristiques des électrodes utilisées est fonction de tests de stimulation opérés sur un patient.

Un inconvénient majeur de ce système réside dans la programmation des motifs de stimulation pour pouvoir imposer une suite de mouvements à un membre du corps humain par les séquences de stimulation électrique. Une liste de motifs de mouvements doit être établie afin de permettre par la suite de combiner certains de ces motifs pour bouger le membre souhaité.

Pour la création de cette liste, une première paire d'électrodes reçoit une première séquence de stimulation pour opérer une contraction d'un muscle. Ensuite de quoi, il est attribué une identification à la première séquence générée. Une seconde séquence de stimulation est fournie à d'autres électrodes pour contracter un autre muscle. Ensuite de quoi une identification est attribuée à la seconde séquence. Cet enregistrement de toutes les séquences de stimulation sur un support d'enregistrement pour la création de cette liste nécessite trop de temps, ce qui est un inconvénient.

Le but que se propose de résoudre l'invention consiste à faciliter et accélérer la programmation des séquences de données de stimulation électrique complexes dans une installation de programmation disposant d'une interface utilisateur graphique pour pallier aux inconvénients des procédés et dispositifs de l'art antérieur.

Un autre but que se propose de résoudre l'invention consiste à enregistrer des séquences de données de stimulation complexes sur un support de mémorisation dans un espace mémoire réduit pour être interprétées dans un appareil de stimulation électrique en palliant les inconvénients des dispositifs et procédés de l'art antérieur.

Ces buts, ainsi que d'autres sont atteints grâce au procédé de programmation de données de stimulation électrique pour un appareil de stimulation électrique neuromusculaire ou fonctionnelle dans une installation de programmation décrit ci-devant, le procédé se caractérise en ce qu'il comprend les étapes de :
- composer une séquence de données de stimulation électrique avec des blocs unitaires de fonctions de stimulation pour au moins un canal de stimulation d'un appareil de stimulation électrique neuromusculaire ou fonctionnelle en plaçant successivement dans une fenêtre principale de l'interface utilisateur graphique de la station d'ordinateur des icônes représentatives chacune de la fonction d'un bloc unitaire correspondant, les icônes étant tirées d'une fenêtre de bibliothèque de blocs unitaires de fonctions de stimulation, et des données de configuration étant appliquées à certains blocs placés dans la séquence,
- traduire dans une unité centrale de traitement de la station d'ordinateur la séquence de données de stimulation composée d'une série de blocs unitaires de fonctions de stimulation configurés en une séquence de données de stimulation codée, dans laquelle chaque bloc unitaire de fonctions configuré est défini par au moins un numéro d'identification spécifique,
- transférer la séquence de données de stimulation codée à destination du dispositif de lecture et d'écriture, et
- commander l'enregistrement de la séquence de données de stimulation codée sur le support de mémorisation placé dans ledit dispositif de lecture et d'écriture.

Un avantage du procédé est de pouvoir créer une ou plusieurs séquences de données de stimulation sur l'interface graphique de la station d'ordinateur pour qu'un programmeur puisse rapidement et facilement établir des séquences adéquates au patient à traiter. Pour ce faire, un assemblage de blocs unitaires de fonctions de stimulation représentés chacun par une icône ou un pictogramme spécifique dans une bibliothèque de l'unité centrale de traitement est réalisé. Pour faciliter cet établissement, une fenêtre principale de l'interface utilisateur graphique est donc prévue dans laquelle une ou plusieurs rangées sont à compléter de blocs unitaires de fonctions de stimulation pour chaque séquence de données de stimulation d'un canal de stimulation correspondant.

Les icônes représentant les blocs unitaires de fonction de stimulation portent des graphismes qui permettent immédiatement et de manière intuitive à un programmeur de construire rapidement les séquences arbitraires de stimulation électrique de chaque canal de stimulation. De plus, un code de couleur peut être appliqué sur les icônes selon qu'elles sont destinées pour un ou plusieurs canaux de stimulation.

Cette suite de blocs unitaires de chaque séquence est ensuite codée pour pouvoir être enregistrée sur un support de mémorisation, qui est avantageusement une carte à puce, inséré dans le dispositif de lecture et d'écriture. Ce dispositif de lecture et d'écriture peut être celui d'un appareil de stimulation électrique neuromusculaire ou fonctionnelle.

Avantageusement, le codage des blocs unitaires consiste à attribuer au moins un numéro d'identification à chaque bloc configuré de manière à n'occuper qu'une place réduite de mémoire dans le support de mémorisation, notamment la carte à puce. Comme la carte n'a enregistré que le numéro d'identification de chaque bloc de la séquence codée, ainsi que des paramètres adaptés dans la station d'ordinateur pour l'exécution desdits blocs unitaires des séquences, l'appareil de stimulation recevant la carte doit avoir un programme de gestion complémentaire à celui de la station d'ordinateur. Ce programme complémentaire lui permet de retrouver les blocs unitaires en fonction de leur numéro d'identification pour la génération des impulsions électriques à fournir aux électrodes de stimulation. La carte, une fois programmée, peut être placée dans n'importe quel appareil de stimulation électrique à programme de gestion complémentaire de manière à pouvoir rapidement changer d'appareil en cas de disfonctionnement du précédent appareil.

Un autre avantage dudit procédé consiste à pouvoir programmer successivement plusieurs supports de mémorisation, tels que des cartes à puce, destinés à être placés chacun dans un appareil de stimulation électrique fonctionnelle. Un des supports est programmé en tant que maître avec des données de synchronisation pour qu'un des appareils de stimulation interconnectés recevant les supports de mémorisation agisse en tant que maître alors que les autres appareils agissent en tant qu'esclave. Le cumul ou la mise en parallèle de plusieurs appareils de stimulation peut être utile lorsqu'il est nécessaire de placer un nombre important d'électrodes de stimulation sur plusieurs muscles à stimuler électriquement lors de la gestion des mouvements de membres du corps humain.

Bien entendu, les supports de mémorisation, insérés chacun dans un des appareils de stimulation interconnectés, pourraient être programmés simultanément. Pour ce faire, plusieurs jeux de séquences de données de stimulation sont composés sur la station d'ordinateur pour tous les appareils de stimulation interconnectés. Dans les jeux de séquences, il doit être spécifié quel appareil doit jouer le rôle de maître par rapport aux autres appareils.

La rapidité d'établissement et d'enregistrement des séquences de données de stimulation électrique par le procédé permet une utilisation rapide des appareils de stimulation électrique fonctionnelle dans les premières phases d'une réhabilitation d'un sujet paralysé ou accidenté.

Ce procédé de programmation est donc utilisé avantageusement pour de multiples applications, telles que la réhabilitation, le développement de neuroprothèses, l'évaluation neurologique d'un patient. De plus, il est également destiné à être utilisé pour des centres hospitaliers, pour des centres de recherche et pour des laboratoires de biologie.

L'appareil de stimulation électrique peut être potentiellement utilisé en combinaison avec des techniques de réparation des nerfs centraux pour rééduquer les voies nerveuses et pour la génération d'un profil de mouvement.

Ces buts, ainsi que d'autres sont également atteints grâce à une installation décrite ci-devant et qui se caractérise en ce que des moyens de mémorisation de l'unité centrale comprennent une bibliothèque de blocs unitaires de fonctions de stimulation représentés chacun par une icône spécifique dans une fenêtre de bibliothèque dans l'interface utilisateur graphique, lesdits blocs pouvant être tirés de la fenêtre de bibliothèque et introduits dans une fenêtre principale de l'interface graphique pour la composition d'au moins une séquence de données de stimulation électrique d'au moins un canal de stimulation d'un appareil de stimulation électrique neuromusculaire ou fonctionnelle, et en ce que des moyens d'introduction de données de la station d'ordinateur permettent de configurer certains blocs unitaires de la séquence et/ou d'adapter des paramètres de stimulation, visualisés dans au moins une fenêtre d'adaptation de paramètres sur l'interface utilisateur graphique, pour l'exécution des blocs unitaires de fonctions de stimulation placés dans la séquence de données de stimulation.

Un avantage de l'installation de stimulation électrique avec un programme d'établissement de séquences de stimulation permet de fournir facilement des cartes à puce personnalisées pour des utilisateurs spécifiques. Les cartes comprennent toutes les données de traitement. Elles peuvent être insérées dans tout appareil de stimulation électrique neuromusculaire ou fonctionnelle à programme de gestion de données de stimulation complémentaire au programme de gestion de la station d'ordinateur.

Toute personne peut programmer facilement et rapidement sur l'interface utilisateur graphique des séquences de stimulation électrique composées de blocs unitaires. Les séquences établies peuvent servir à la génération des impulsions électriques à imposer à sa musculature sans forcément nécessité l'avis d'une personne spécialisée.

Les buts, avantages et caractéristiques du procédé et de l'installation de programmation de données de stimulation pour un appareil de stimulation électrique neuromusculaire ou fonctionnelle apparaîtront mieux dans la description suivante de formes d'exécution illustrées par les dessins sur lesquels :
- La figure 1a représente une installation de programmation de données de stimulation avec un appareil de stimulation relié par câble à une station d'ordinateur,
- la figure 1b représente une installation de programmation de données de stimulation avec deux appareils de stimulation interconnectés reliés par câble à une station d'ordinateur,
- la figure 1c représente une installation de programmation de données de stimulation avec un appareil de stimulation en liaison sans fil à une station d'ordinateur,
- la figure 2 montre schématiquement les blocs électroniques d'une station d'ordinateur reliée à un appareil de stimulation recevant une carte à puce,
- la figure 3 montre schématiquement les blocs électroniques d'un appareil de stimulation électrique recevant une carte à puce programmée par le procédé de programmation selon l'invention,
- la figure 4 montre schématiquement les blocs électroniques composant la carte à puce programmée par le procédé de programmation selon l'invention,
- les figures 5a et 5b montrent deux représentations dans la fenêtre principale de l'interface utilisateur graphique de séquences de données de stimulation composées et des boutons graphiques d'ajustement de différents paramètres,
- la figure 6 montre la fenêtre de bibliothèque de blocs unitaires de fonctions de stimulation représentés chacun par une icône spécifique,
- les figures 7a à 7e montrent les différentes catégories de blocs unitaires de fonctions de stimulation représentés chacun par une icône,
- la figure 8a représente une fenêtre d'adaptation de paramètres des canaux de stimulation, tels que les rampes, les constantes et les variations aléatoires de largeur d'impulsions,
- la figure 8b représente une fenêtre d'édition pour la modification d'une courbe de rampe de largeur d'impulsions liée à la fenêtre de la figure 8a,
- la figure 8c montre un graphique d'une variation linéaire en fonction du temps, à amplitude et à fréquence constantes, de largeur d'impulsions de courant biphasique pour une rampe de largeur d'impulsions vue en figure 8a,
- la figure 9a montre une fenêtre d'adaptation de paramètres, tels que les constantes de fréquence, les constantes d'amplitude d'impulsions par une transition linéaire et la variation aléatoire d'amplitude et/ou de fréquence,
- la figure 9b montre schématiquement un graphique d'une transition linéaire d'amplitude d'impulsions de courant biphasique en fonction du temps,
- la figure 10a montre une fenêtre d'adaptation de paramètres concernant des interactions d'un utilisateur,
- la figure 10b montre une fenêtre de critères d'adaptation d'un type analogique d'interaction d'un utilisateur liée à la fenêtre de la figure 10a,
- la figure 11 montre des variations d'amplitude d'impulsions de stimulation de canaux de stimulation sur la base des mesures de capteurs de commande pour l'interaction d'un utilisateur,
- la figure 12 montre une fenêtre d'adaptation de paramètres qui concerne l'inscription de textes à visualiser sur un écran d'un appareil de stimulation, et
- les figures 13a et 13b montrent deux graphiques de largeur d'impulsions en fonction du temps de deux canaux de stimulation, ainsi que les deux séquences correspondantes réalisées sur la fenêtre principale.

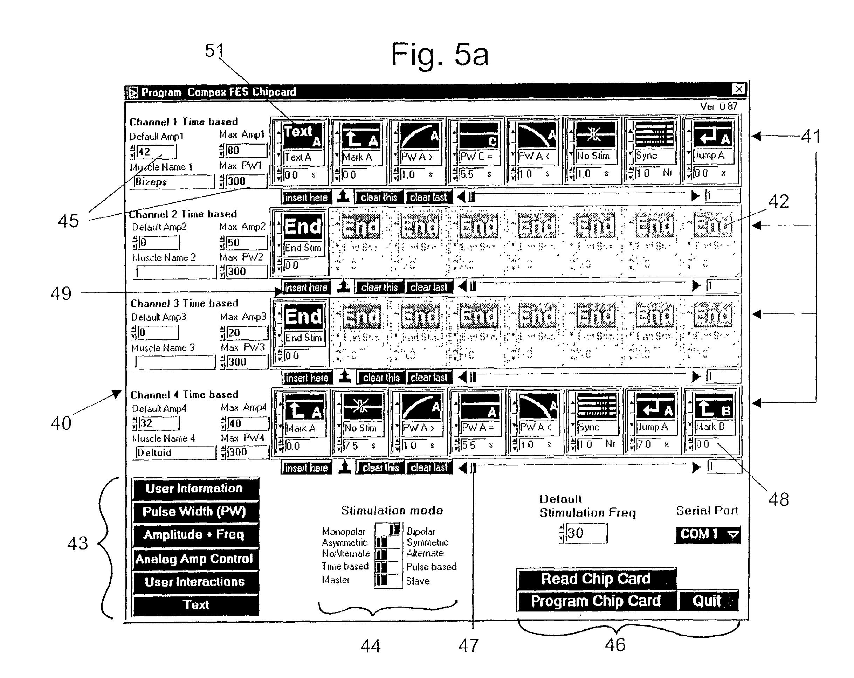

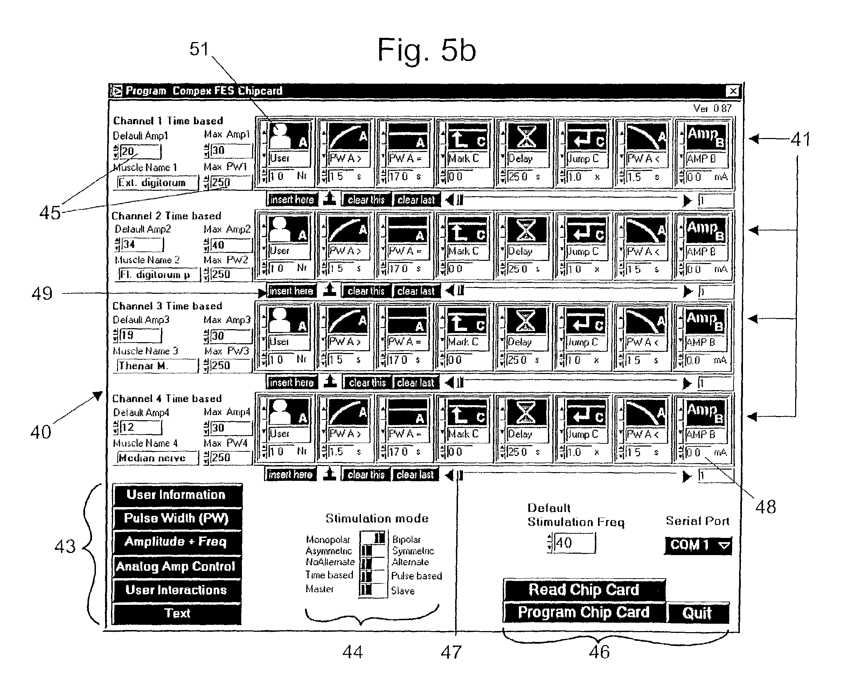

La description suivante pour la programmation de données de stimulation sera basée de préférence sur une installation de programmation qui comprend une station de travail à programme de gestion de données de stimulation et à interface utilisateur graphique, au moins un dispositif de lecture et d'écriture d'un appareil de stimulation électrique neuromusculaire ou fonctionnelle en communication avec la station d'ordinateur, et au moins un support de mémorisation, tel qu'une carte à puce, à insérer dans le dispositif de lecture et d'écriture pour mettre en mémoire des données de stimulation électrique personnalisées. Les composants électroniques des éléments de l'installation ne seront pas décrits en détails car ils font partie des connaissances générales d'un homme du métier dans ce domaine technique. Par contre, les étapes du procédé de programmation de données de stimulation électrique à l'aide de l'installation seront détaillées et visualisées en référence aux figures 5 à 13 dans la suite de la description.

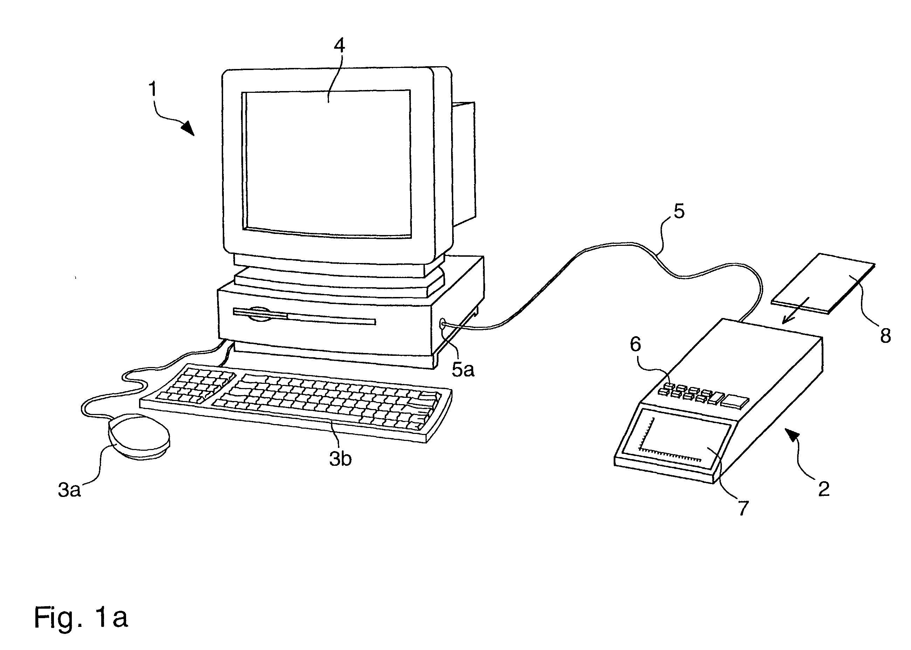

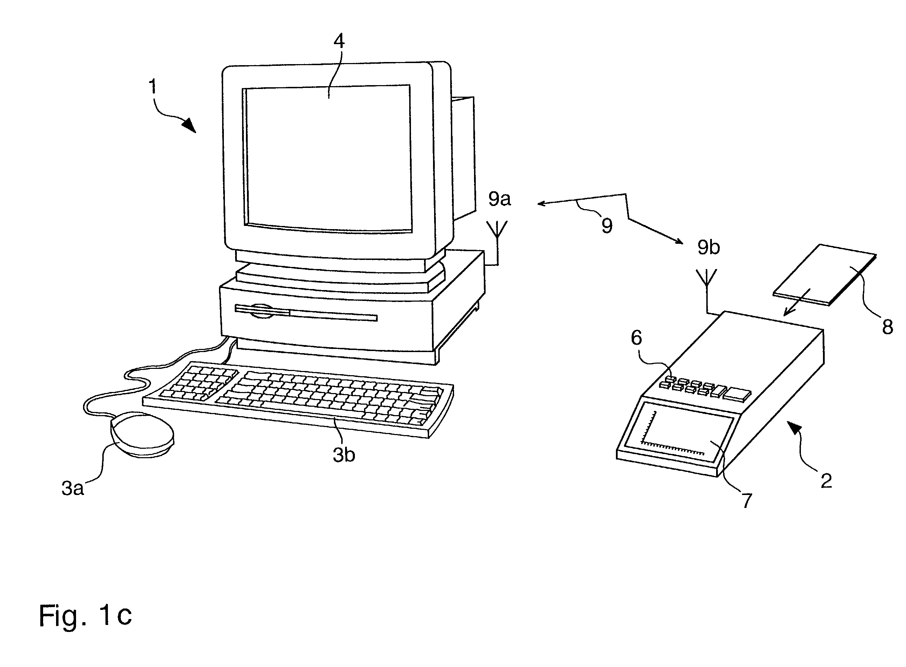

En référence à la figure 1a, l'installation de programmation de données de stimulation électrique est constituée d'une station d'ordinateur 1 à interface utilisateur graphique, d'un câble électrique 5 connecté à une sortie 5a de la station d'ordinateur 1 et à une entrée d'un appareil portatif de stimulation électrique neuromusculaire ou fonctionnelle 2, et d'au moins une carte à puce 8. Ladite carte, en tant que support de mémorisation, est insérée dans un dispositif de lecture et d'écriture de l'appareil pour recevoir des séquences de données de stimulation codées de la station d'ordinateur 1. Chaque séquence établie pour chaque canal de stimulation est enregistrée en des positions mémoires déterminées dans la carte à puce 8.

Un programme de gestion de données est installé dans la station d'ordinateur 1 pour pouvoir notamment composer et traduire des séquences de données de stimulation. Ladite station d'ordinateur 1 est composée classiquement de moyens d'introduction de données, qui sont représentés par une souris 3a et par un clavier 3b reliés à des entrées correspondantes de la station, et d'un écran de visualisation 4 de l'interface utilisateur graphique. L'interface utilisateur graphique de la station d'ordinateur 1 permet donc à un programmeur de définir de manière aisée et intuitive des séquences arbitraires de données de stimulation. Lesdites séquences sont définies pour chaque canal de stimulation d'un appareil de stimulation électrique fonctionnelle dans une fenêtre principale de l'interface. Cette fenêtre principale, ainsi que les autres fenêtres reliées à la fenêtre principale, seront expliquées en détail à partir des figures 5a et 5b.

Le programmeur compose donc les séquences de données de stimulation sur l'écran 4 de l'interface graphique de la station d'ordinateur 1 en fonction des canaux de stimulation d'un appareil de stimulation électrique 2. Ces séquences établies sont traduites en des séquences de stimulation codées. Les séquences codées sont ensuite envoyées par voie sérielle les unes à la suite des autres au dispositif de lecture et d'écriture de l'appareil enclenché pour les enregistrer sur la carte à puce 8. L'ordre des séquences transmises est fonction par exemple du numéro de chaque canal de stimulation.

Il est à noter que le codage des séquences de données de stimulation permet de réduire la taille des données à enregistrer sur la carte à puce pour qu'elles n'occupent qu'un espace réduit de mémoire de la carte.

L'appareil portatif de stimulation électrique neuromusculaire ou fonctionnelle 2 comprend un écran de visualisation 7 sur lequel des graphiques ou des valeurs ou des textes peuvent être affichés selon la programmation, par exemple, des séquences de stimulation sur la carte à puce 8. L'appareil comprend encore des boutons 6 de commande ou de réglage de paramètres de stimulation, des sorties pour connecter des électrodes de stimulation et des entrées pour des mesures de capteurs ou des commandes d'actionneurs pour les interactions d'un utilisateur, non représentés sur la figure 1a. Dans une première phase du procédé de programmation, uniquement le dispositif de lecture et d'écriture de l'appareil est utilisé pour enregistrer des séquences de données de stimulation électrique sur la carte à puce 8.

Dans cette première forme d'exécution de l'installation, l'appareil de stimulation électrique 2 est de préférence l'appareil dénommé Compex2 fourni par l'entreprise Compex S.A. sise à Ecublens en Suisse. Cet appareil de stimulation électrique 2 comprend notamment quatre canaux de stimulation et doit être muni d'un programme de gestion de données complémentaire au programme de la station d'ordinateur 1. Toutefois, tout autre appareil de stimulation électrique peut bien évidemment être utilisé pour autant qu'il comprenne un dispositif de lecture et d'écriture d'un support amovible de mémorisation 8 et un programme de gestion de données de stimulation complémentaire au programme de la station d'ordinateur 1.

De la station, les séquences de données sont traduites par ledit programme de gestion en des séquences codées à enregistrer sur la carte. Après cette étape, l'appareil de stimulation est mis en fonction pour interpréter ou traduire inversement les séquences codées de la carte à puce à l'aide du programme de gestion complémentaire. Le programme de gestion de données de stimulation est notamment relatif aux paramètres de stimulation pour faciliter l'introduction desdites données de stimulation.

De plus lors de l'utilisation de l'appareil de stimulation avec le support de mémorisation programmé, une adaptation des paramètres de stimulation peut être réalisée suite à des mesures effectuées par des capteurs placés au niveau des muscles stimulés électriquement ou au niveau des muscles à contraction volontaire. Ces mesures permettent une gestion optimale du mouvement d'un muscle stimulé électriquement. Certains capteurs ou actionneurs servent par contre comme dispositif d'interaction d'un utilisateur, lors de l'exécution des séquences mémorisées sur la carte, pour la commande d'actionnement de l'appareil de stimulation.

L'appareil de stimulation électrique fonctionnelle est de préférence une neuroprothèse destinée notamment à la réhabilitation de sujets handicapés ou accidentés afin de leur permettre de retrouver des fonctions corporelles perdues ou manquantes. Cette neuroprothèse leur fournit une indépendance de leurs mouvements sans nécessité l'aide d'autres personnes dans leurs activités quotidiennes. La carte à puce 8 est donc programmée selon le procédé de l'invention avec des séquences de données de stimulation codées qui sont, dans ce cas, complexes pour pouvoir être utilisée dans une telle neuroprothèse.

A titre informatif pour l'utilisation d'appareils de stimulation électrique, il faut savoir que pour la contraction des muscles, des trains d'impulsions électriques sont appliqués par l'intermédiaire d'électrodes posées sur des points moteurs des nerfs à innerver. La phase de contraction desdits muscles est réalisée par plusieurs impulsions qui se répètent dans un intervalle de temps prédéfini. La fréquence desdites impulsions est de l'ordre de quelques Hertz jusqu'à environ 100 Hz. Une fréquence élevée empêche les muscles contractés de revenir dans une position décontractée. Habituellement, il peut être préconisé d'alterner des suites d'impulsions de contraction avec des pauses pour permettre justement aux muscles innervés par les impulsions électriques de se décontracter.

Le réglage de l'amplitude du courant ou de la tension de chaque impulsion électrique permet de choisir le nombre de fibres musculaires à recruter lors de la stimulation. Plus l'amplitude du courant ou de la tension des impulsions électriques est élevée, et plus le nombre de fibres recrutées est élevé entre une valeur minimum et maximum d'amplitude de courant ou de tension.

On peut retracer des portions de la relation entre le couple de force et l'amplitude du courant de stimulation. Par exemple, une portion concerne une forte variation du couple avec l'accroissement de l'amplitude du courant. Une autre portion, notamment dès 60 mA, définit que le couple de force atteint un plafond, ce qui signifie que toutes les fibres ont été recrutées. Une relation peut être également faite entre le couple et la durée des impulsions de courant imposées.

Dans ce domaine médical, l'utilisation de courtes impulsions électriques pour générer une contraction musculaire est appelée stimulation électrique fonctionnelle. Dans la terminologie anglaise, l'abréviation FES (Functional Electrical Stimulation) est utilisée. La contraction musculaire est opérée en générant des potentiels d'action dans les nerfs moteurs afin de causer ladite contraction. Il est d'une importance primordiale que le sujet ait des neurones motrices secondaires intactes pour pouvoir lui imposer des stimulations électriques susceptibles d'agir sur la contraction musculaire.

Les muscles peuvent être stimulés en utilisant des impulsions de tension ou de courant monophasiques ou biphasiques avec, si possible, une alternance de l'anode et de la cathode durant la stimulation.

Comme l'emploi d'impulsions de courant monophasique sur la peau a le désavantage d'induire des risques de brûlure, il est préconisé de générer habituellement des impulsions de courant biphasiques de manière que la moyenne du courant soit nulle. Ce type d'impulsions biphasiques résout le phénomène de polarisation des fibres. De plus, pour avoir une force de contraction la plus haute possible, il est préféré de générer des impulsions rectangulaires biphasiques dans lesquelles la phase positive est équivalente à la phase négative. Cette impulsion électrique biphasique rectangulaire permet avec la même intensité d'avoir un meilleur recrutement spatial des unités motrices. II doit donc être tenu compte de la forme des impulsions à imposer aux muscles spécifiquement pour l'adaptation des mouvements d'un membre portant une neuroprothèse.

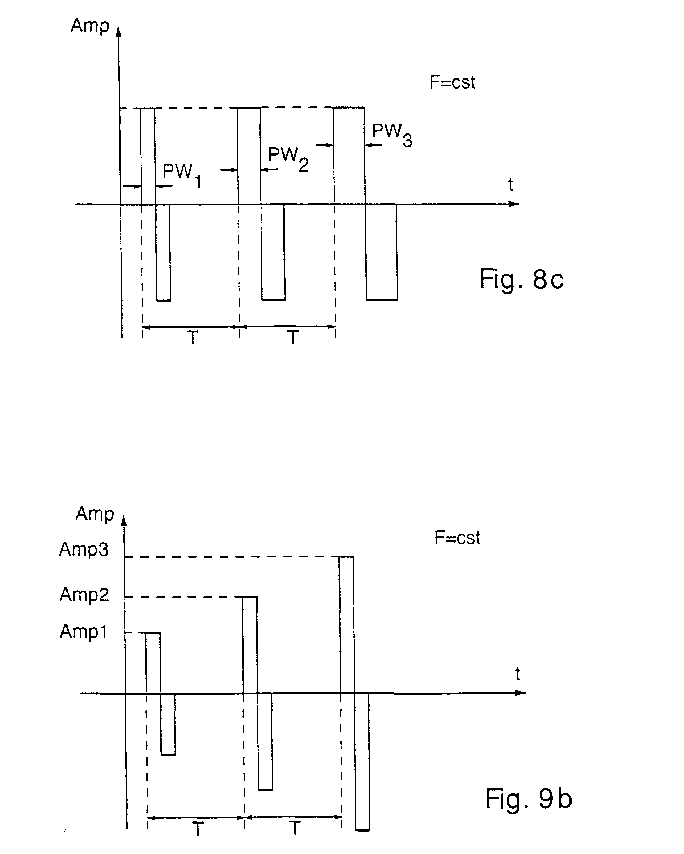

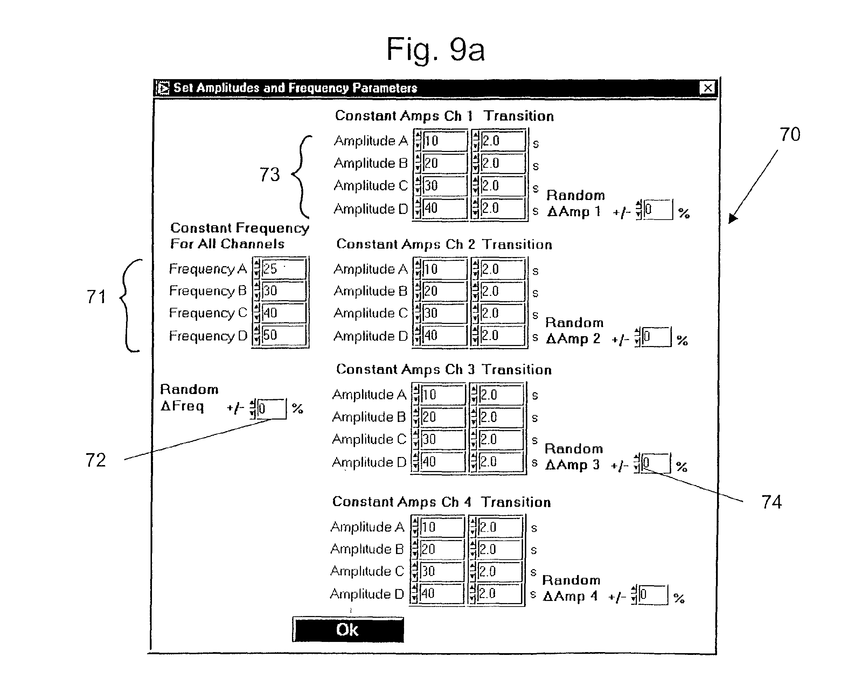

Des rampes de progression de l'amplitude des impulsions peuvent être également programmées de manière à sélectionner graduellement les fibres musculaires ou nerveuses stimulées électriquement plutôt que d'exciter toutes les unités motrices abruptement. A titre illustratif, un graphique d'une variation linéaire d'amplitude en fonction du temps est représenté en figure 9b. Les impulsions de courant à fréquence constante sont biphasiques, symétriques et non alternées. L'amplitude des impulsions, qui peut être choisie jusqu'à une valeur maximale de 100 mA, varie d'une valeur AMP1 à une valeur AMP2 et finalement à une valeur AMP3 dans une période de transition définie. L'introduction des constantes de variation d'amplitude sera montrée en référence à la figure 9a dans la suite de la description.

Plutôt que ces rampes d'amplitude, une autre méthode consiste à moduler la durée des impulsions de courant biphasiques. A titre illustratif, un graphique d'une variation de largeur d'impulsions en fonction du temps est représenté en figure 8c. Les largeurs d'impulsions sont variées d'une valeur PW1 à une valeur PW2 et finalement à une valeur PW3. Il est plus important de spécifier la durée de phase de chaque impulsion plutôt que la durée totale de l'impulsion.

La stimulation des points moteurs peut être réalisée par des électrodes de surface ou des électrodes sous-cutanées qui sont capables de délivrer des charges électriques à des points moteurs de manière plus sélective que les électrodes de surface. Par contre, lesdites électrodes de surface peuvent être placées rapidement sans intervention chirurgicale sur la peau d'un patient ce qui constitue un net avantage dans le cas de la réhabilitation d'un patient.

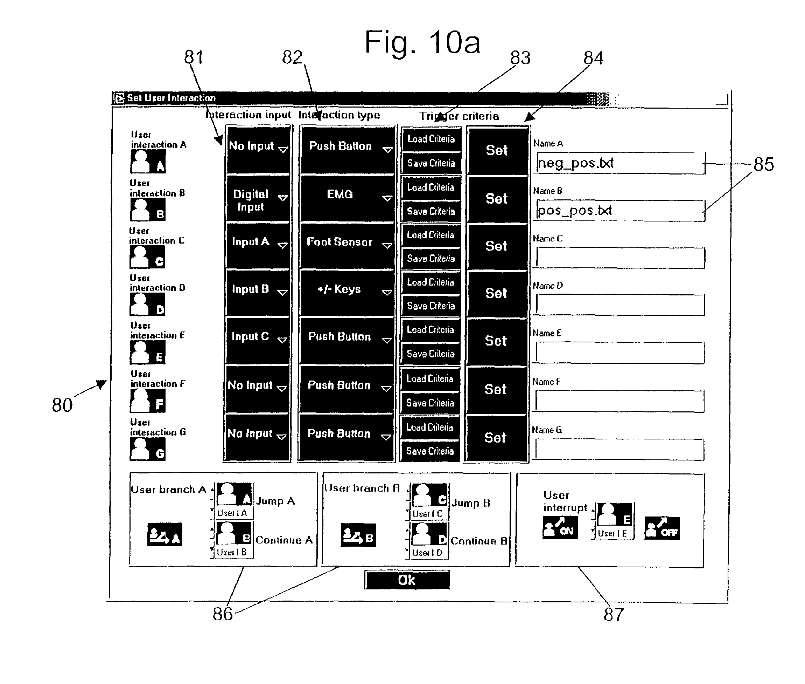

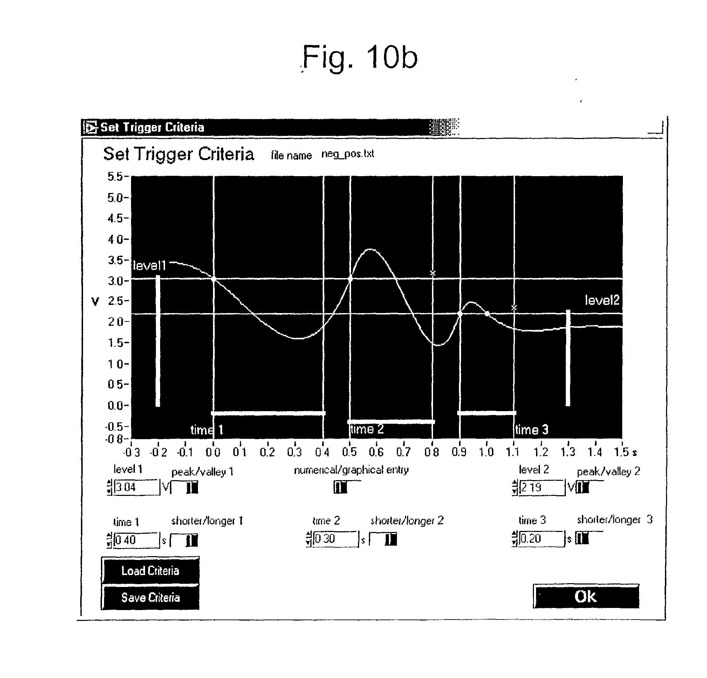

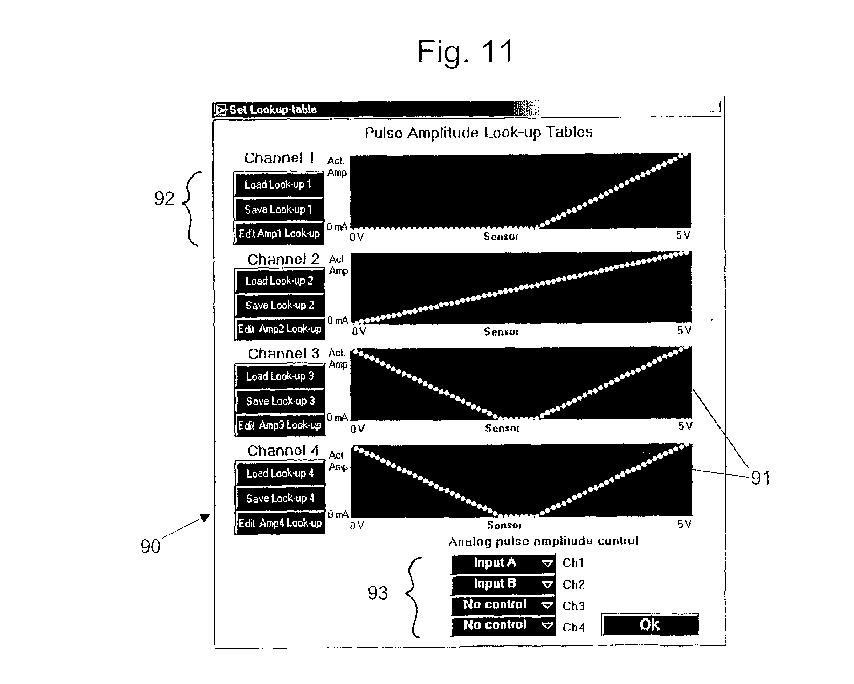

Pour des patients handicapés, par exemple pour des tétraplégiques, des appareils portables de stimulation électrique sont utilisés pour activer leurs muscles rendus inactifs. Un tel appareil de stimulation peut être employé par exemple pour l'action des muscles d'une main pour la saisie d'objets. Un capteur de position ou un capteur électromyographique (EMG) placé sur l'épaule de ladite personne, en tant que dispositif d'interaction, commande la génération des trains d'impulsions électriques de la neuroprothèse. Une fenêtre d'adaptation de paramètres est prévue pour choisir le type d'interaction d'un utilisateur et pour adapter les mesures d'un capteur de type analogique comme représenté aux figures 10a, 10b et 11.

De telles neuroprothèses recevant un support de mémorisation, dont les séquences de données de stimulation codées ont été enregistrées dans l'installation de programmation, peuvent être également utilisées pour ordonner les séquences de stimulation électrique des muscles de jambes pour permettre la marche du sujet.

Pour de plus amples informations au sujet des neuroprothèses, le lecteur peut se référer à l'article intitulé "ETHZ-Paracare Grasping and Walking Neuroprostheses" de M.R. Popovic, T. Keller, I. Pappas, V. Dietz et M. Morari publié dans IEEE Engineering in Medicine and Biology Magazine en mars 2000 sous la référence EMB99-FJD3.

Avec cette installation de programmation, il peut être prévu dans une phase préliminaire de placer le support de mémorisation dans le dispositif de lecture et d'écriture pour être lu. Ensuite, la station d'ordinateur ordonne la lecture du support, qui comprend des séquences de données de stimulation codées préalablement mémorisées.

La commande de lecture permet de visualiser sur la fenêtre principale de l'interface utilisateur graphique les séquences de données de stimulation mémorisées sur la carte. De même, les données de paramètres adaptés dans des fenêtres d'adaptation peuvent être également visualisées pour vérifier et/ou modifier ces données. La station d'ordinateur peut mémoriser les données de stimulation lues de la carte.

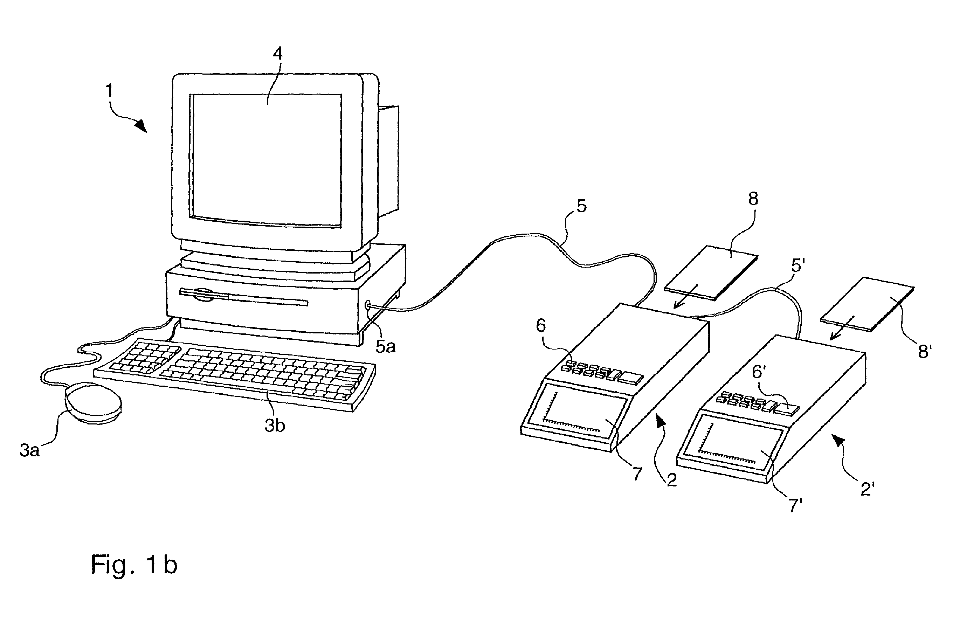

En référence à la figure 1b, une seconde forme d'exécution de l'installation notamment pour la programmation de plusieurs cartes à puces 8 et 8' est montrée. Les mêmes éléments de cette figure 1b portent des signes de référence identiques à ceux de la figure 1a.

Il est à noter que les cartes à puce 8 et 8' peuvent être préprogrammées pour être utilisées dans plusieurs appareils de stimulation électrique fonctionnelle interconnectés 2 et 2'.

Dans cette figure 1b, la station d'ordinateur 1, qui comprend notamment un écran de visualisation 4 des données de stimulation électrique et des moyens d'introduction de données 3a et 3b, est reliée depuis une sortie 5a de la station par un câble électrique 5 à un premier appareil de stimulation électrique 2. Ce premier appareil de stimulation électrique 2 est lui-même relié par un câble électrique 5' à un second appareil de stimulation électrique 2'.

Les câbles électriques ou bus de données 5 et 5' comprennent plusieurs fils électriques pour le transfert des données de stimulation entre la station d'ordinateur et les appareils de stimulation interconnectés. Ce transfert de données n'est donc plus réalisé par voie sérielle comme dans la première forme d'exécution, car la communication par voie sérielle n'est possible qu'entre deux dispositifs interconnectés.

Chaque appareil a un écran de visualisation 7 et 7' sur lequel sont affichés des valeurs d'amplitude, de fréquence et/ou de largeur d'impulsions, des textes ou des graphiques relatifs à la stimulation électrique. Une première carte à puce 8 est insérée dans le dispositif de lecture et d'écriture du premier appareil 2, et une seconde carte à puce 8' est insérée dans le dispositif de lecture et d'écriture du second appareil 2'.

Dans une première étape du procédé de programmation, des séquences de données de stimulation sont composées à l'aide de l'interface utilisateur graphique de la station d'ordinateur 1 pour tous les canaux de stimulation des appareils de stimulation électrique 2 et 2'. Ces séquences sont traduites en des séquences de données de stimulation codées qui comprennent chacune en plus des indications des paramètres adaptés pour chaque canal, ainsi que pour chaque appareil.

Ces séquences codées sont envoyées par le bus de données 5 en direction du dispositif de lecture et d'écriture du premier appareil où les séquences codées destinées à cet appareil sont enregistrées sur la carte à puce 8. Les autres séquences codées sont transmises par l'intermédiaire d'un autre bus de données 5' entre le premier appareil 2 et le second appareil 2' au dispositif de lecture et d'écriture du second appareil 2' où ces séquences codées sont enregistrées sur la carte à puce 8'.

Lors de l'établissement des séquences de données de stimulation sur la station d'ordinateur, une sélection entre le mode maître et le mode esclave peut être déterminée pour chaque jeu de séquences de données de stimulation destiné à chaque appareil. Par exemple, la carte à puce 8 peut enregistrer le mode maître pour imposer au premier appareil de stimulation électrique 2 de jouer le rôle de maître pour synchroniser tous les canaux de stimulation des autres appareils interconnectés, qui doivent jouer le rôle d'esclave.

Il est à noter que la programmation des séquences de stimulation codées pour plusieurs cartes à puce est réalisée plus avantageusement de manière successive avec un unique dispositif de lecture et d'écriture dans l'installation de programmation, comme montré à la figure 1a. Les cartes à puce 8, une fois programmées avec le mode maître ou le mode esclave, sont insérées par la suite dans plusieurs appareils de stimulation interconnectés. Le premier des appareils de stimulation ayant la carte maître joue le rôle de maître pour synchroniser tous les autres appareils interconnectés qui jouent de ce fait le rôle d'esclave.

Cette possibilité de programmer sur la carte à puce les séquences avec le mode maître ou le mode esclave permet avantageusement de cumuler ou mettre en parallèle plusieurs appareils de stimulation interconnectés synchronisés par l'appareil maître. Ces appareils interconnectés servent essentiellement comme neuroprothèse pour ordonner le mouvement de membres du corps par le biais de plusieurs paires d'électrodes de stimulation placées sur des points moteurs de muscles à stimuler électriquement.

Ces appareils de stimulation électrique peuvent permettre en combinaison avec les techniques de réparation des nerfs centraux de rééduquer les voies nerveuses ou d'établir des profils de mouvement.

En référence à la figure 1c, une troisième forme d'exécution de l'installation de programmation est montrée dans laquelle la transmission des données de stimulation 9 entre la station d'ordinateur 1 et l'appareil de stimulation 2 se fait par des moyens d'émission et de réception de signaux sans fil. Ces moyens d'émission et de réception sont représentés uniquement par une antenne 9a sur la station d'ordinateur et une antenne 9b sur l'appareil de stimulation 2, mais ils ne seront pas expliqués en détail car ils font partie des connaissances générales d'un homme du métier.

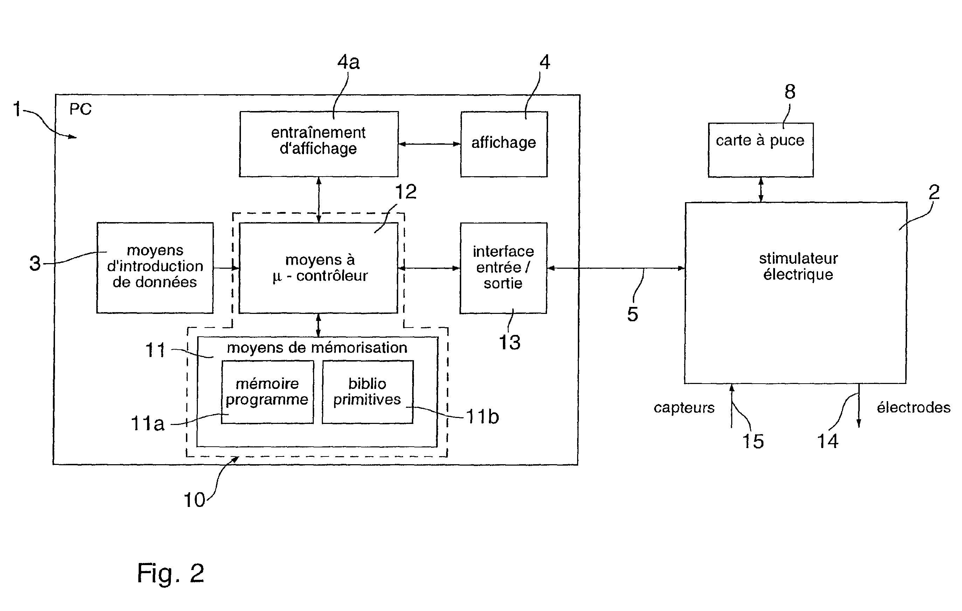

En figure 2, les blocs électroniques de la station d'ordinateur 1 sont représentés pour la mise en oeuvre du procédé de programmation des séquences de données de stimulation selon l'invention.

La station d'ordinateur 1, qui peut être une station de travail personnelle, comprend une unité centrale de traitement des données 10. L'unité est constituée principalement de moyens à microcontrôleurs 12 pour le traitement et la gestion de toutes les données de stimulation, et des moyens de mémorisation 11. Ces moyens de mémorisation sont composés notamment d'une mémoire 11a de programme de gestion des données de stimulation et d'une mémoire 11b d'une bibliothèque de blocs unitaires de fonctions de stimulation. Les moyens de mémorisation 11 comprennent également une mémoire, non représentée sur la figure 2, pour le stockage de données de stimulation lors de la composition des séquences de données de stimulation ou la lecture des séquences de données de stimulation d'un support de mémorisation préalablement programmé.

Le programme stocké dans la mémoire 11a pour permettre le traitement des données de stimulation des moyens à microcontrôleurs 12, ainsi que la bibliothèque de blocs unitaires de fonctions de stimulation 11b sont par exemple placés sur le disque dur de la station d'ordinateur. Cela permet ainsi de préserver le programme de gestion de données de stimulation même lors d'une interruption d'alimentation électrique de la station d'ordinateur 1.

La station d'ordinateur 1 comprend encore des moyens d'introduction de données 3, qui ont déjà été montrés aux figures 1a à 1c, une interface utilisateur graphique liée à l'unité centrale de traitement 10 et une interface d'entrée et de sortie de signaux de données 13 reliée aux moyens à microcontrôleurs 12. L'interface graphique comprend notamment un bloc d'entraînement d'affichage 4a géré par l'unité centrale et un dispositif d'affichage 4. Les signaux de données de l'interface d'entrée et de sortie 13 sont transmis par voie sérielle 5 vers l'appareil de stimulation électrique 2 pour l'enregistrement des séquences de données de stimulation codées sur la carte à puce 8. Les séquences codées sont placées les unes à la suite des autres pour la transmission sérielle entre la station d'ordinateur et le dispositif de lecture et d'écriture de l'appareil de stimulation. Ledit appareil comprend en outre des entrées de données de capteurs 15 et des sorties de connexion d'électrodes de stimulation 14.

Les fenêtres apparaissant sur le dispositif d'affichage 4 de la station d'ordinateur 1 peuvent être sélectionnées par exemple par un pointeur d'une souris des moyens d'introduction de données 3. Des valeurs ou des textes peuvent être également introduits par le clavier des moyens d'introduction de données 3 en des positions déterminées des fenêtres de composition des séquences de données de stimulation. Cette programmation des séquences de données de stimulation sur l'interface utilisateur graphique nécessite d'installer le pilote d'exécution de LabView (version 5.1) par exemple sur une station de travail personnelle 1.

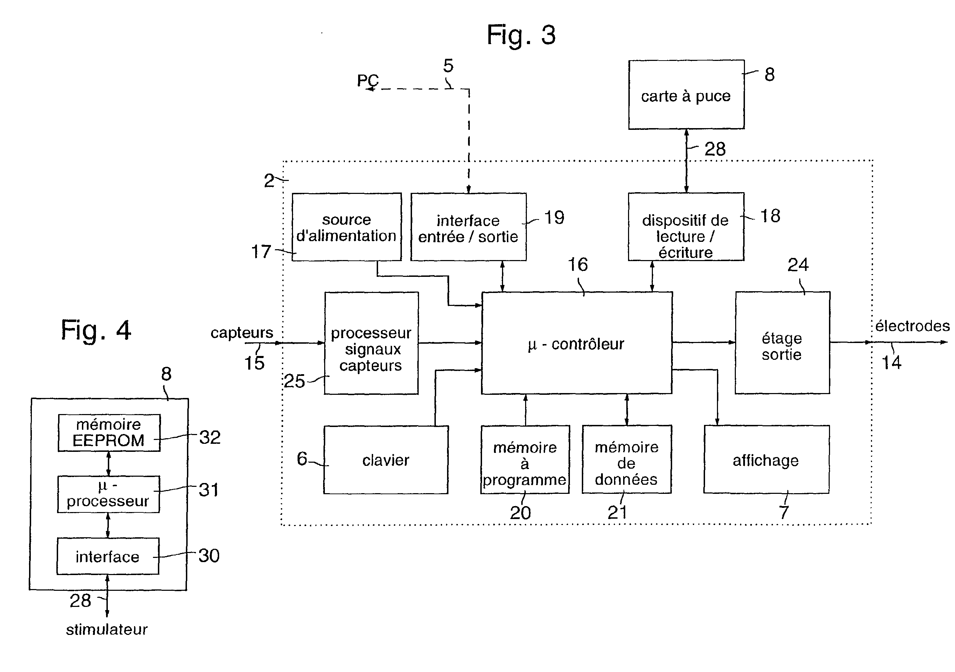

A la figure 3, les blocs électroniques de l'appareil de stimulation 2, servant dans une première phase d'intermédiaire à l'enregistrement de séquences de données de stimulation codées sur la carte à puce 8, sont représentés.

L'appareil portable de stimulation électrique 2 est composé d'une interface d'entrée et de sortie 19 de signaux de stimulation électrique en connexion avec la station d'ordinateur par la voie sérielle 5, d'un microcontrôleur 16 relié à l'interface et traitant les séquences de données de stimulation, d'une mémoire à programme de gestion des données de stimulation 20 liée au microcontrôleur 16, d'une mémoire de stockage de données 21 du microcontrôleur 16, d'un clavier 6 d'introduction de paramètres de stimulation ou de mode de fonctionnement pour le microcontrôleur 16 et d'un dispositif de lecture et d'écriture 18 recevant une carte à puce 8 pour l'enregistrement des séquences de données de stimulation codées. Les blocs électroniques de l'appareil de stimulation 2 sont alimentés par une source d'énergie 17 qui peut être une batterie rechargeable.

Les données traitées dans le microcontrôleur 16 lors de la mise en fonction de l'appareil de stimulation 2 peuvent être affichées sur un dispositif d'affichage 7 selon une sélection opérée sur un des boutons du clavier 6.

Pendant les étapes du procédé de programmation, l'appareil de stimulation n'est enclenché que pour permettre au dispositif de lecture et d'écriture 18 de transférer les séquences de données de stimulation codées provenant de la station d'ordinateur sur la carte à puce 8 pour qu'elles soient mémorisées. Par contre, après ces étapes de programmation, la carte à puce 8 est introduite dans un appareil de stimulation 2 qui comprend un programme de gestion de données de stimulation complémentaire à celui de la station d'ordinateur. Ce programme de gestion, qui est un logiciel écrit dans le langage assembleur, est enregistré dans la mémoire à programme 20 pour permettre au microcontrôleur 16 de traduire les séquences codées lues de la carte à puce 8. Chaque bloc unitaire des séquences codées, enregistré sur la carte sous au moins un numéro d'identification spécifique, est reconnu par le microcontrôleur 16, ainsi que les paramètres adaptés liés aux blocs unitaires de fonctions de stimulation. Après la lecture de la carte, des trains d'impulsions électriques sont générés par l'étage de sortie 24 à au moins une paire d'électrodes de stimulation 14 d'un canal de stimulation. Les séquences codées de la carte à puce 8 peuvent être enregistrées dans une mémoire 21 lors de la mise en marche de l'appareil de stimulation.

L'appareil portable de stimulation électrique fonctionnelle 2 comprend également des entrées pour des signaux de capteurs 15 ou d'actionneurs. Les signaux de mesure sont fournis à un processeur 25 de signaux des capteurs 15 ou des actionneurs afin que le microcontrôleur 16 traite les données fournies par le processeur 25. Dans le traitement de ces données fournies par le processeur 25, le microcontrôleur prend en compte sur la base du programme de gestion de données de stimulation et des séquences de données de stimulation codées de la carte à puce 8 quelles variations ou commandes doivent être opérées pour la génération des trains d'impulsions électriques de l'étage de sortie 24. Les mesures faites par les capteurs ou les actionneurs peuvent imposer un changement d'amplitude des impulsions électriques afin de moduler la force ou le couple de force d'une neuroprothèse pour la saisie d'objets par exemple.

En référence à la figure 4, la carte à puce 8, qui est bien connue dans ce domaine technique, comprend une interface 30 d'entrée et de sortie des données de stimulation par une connexion 28 avec le dispositif de lecture et d'écriture de l'appareil de stimulation électrique. Les séquences de données de stimulation sont transmises de l'interface 30 au microprocesseur 31 et à la mémoire EEPROM 32. Les adresses des positions de la mémoire 32 où doivent être stockées les diverses données des séquences sont gérées par le microprocesseur 31.

Les séquences de stimulation programmées par l'utilisateur et les agencements de commande peuvent être enregistrés entièrement sur la carte à puce 8 qui peut être lue et exécutée par n'importe quel appareil de stimulation par exemple du type Compex2. L'information des séquences entières est sur la carte à puce et non dans l'appareil de stimulation, ce qui simplifie son maniement. Pour tout changement de programme avec le même appareil de stimulation, il suffit de changer uniquement la carte à puce.

L'espace mémoire de la carte à puce 8 étant relativement faible (2 kbytes), les séquences sont codées pour y être mémorisées et placées en des positions déterminées de la mémoire EEPROM 32. Au moins un numéro d'identification est attribué à chaque bloc unitaire de fonctions de stimulation configuré placé dans chaque séquence de données de stimulation. Les numéros d'identification de blocs configurés sont enregistrés sur la carte à puce en des positions déterminées afin que les séquences codées n'occupent qu'un espace réduit dans la mémoire de la carte.

La moitié de la mémoire de la carte à puce est utilisée pour enregistrer notamment des données de l'utilisateur, des paramètres de stimulation, des rampes, des constantes et d'autres données. Les positions mémoires correspondantes sont définies par les adresses 6000 à 63FF en hexadécimal.

L'espace mémoire restant de la carte à puce est utilisé pour enregistrer les séquences de blocs unitaires, c'est-à-dire les numéros d'identification des blocs unitaires configurés pour chaque canal. Les positions mémoires correspondantes sont définies par les adresses 6400 à 67FF en hexadécimal.

L'appareil de stimulation recevant la carte programmée sait retrouver la fonction de chaque bloc unitaire configuré et mémorisé grâce à un programme de gestion de données de stimulation complémentaire à celui de la station d'ordinateur.

Comme la programmation de la carte est réalisée avec un dispositif portable de lecture et d'écriture connecté à une station d'ordinateur à programme de gestion de données de stimulation, cela permet un échange, une modification ou une mise à jour de données de stimulation entre un thérapeute et un patient lors d'un traitement.

Les traitements à l'aide de ces appareils de stimulation électrique neuromusculaire avec leur carte à programme de traitement personnalisé peuvent se faire au logis du patient. Toutefois, un suivi du traitement doit être fait par un thérapeute afin qu'il puisse constater l'évolution de traitement des parties corporelles accidentées ou affaiblies grâce au stimulateur ou à la prothèse de stimulation électrique.

Le procédé de programmation de données de stimulation électrique sera maintenant expliqué sur la base, tout d'abord, des figures 5a et 5b qui représentent chacune une fenêtre principale de l'interface utilisateur graphique avec des exemples différents de séquences de données de stimulation établies. Dans la figure 5a, des séquences de données de stimulation ont été composées pour le premier et le quatrième canal de stimulation, alors que dans la figure 5b des séquences de données de stimulation ont été composées pour tous les canaux de manière similaire.

La fenêtre principale 40, qui apparaît sur l'écran de l'interface utilisateur graphique lors de la mise en route de la station d'ordinateur avec le programme de gestion de données de stimulation, permet de composer plusieurs séquences de données de stimulation pour plusieurs canaux de stimulation d'un appareil de stimulation électrique. Dans le cas présent, uniquement quatre séquences de données de stimulation sont visibles et suffisantes pour les quatre canaux de l'appareil de stimulation électrique, tel que l'appareil Compex2.

Chaque séquence de données de stimulation est disposée dans une rangée horizontale 41, alors que chaque canal de stimulation avec sa séquence est représenté verticalement sur la fenêtre principale.

Les rangées de séquences de données de stimulation comprennent chacune un certain nombre de cases vides 42, par exemple 254 cases, dont uniquement 8 sont visibles sur la fenêtre principale 40. Toutes les cases de chaque séquence peuvent toutefois être visionnées en déplaçant un curseur graphique 47 correspondant à l'aide d'un pointeur actionné par la souris d'ordinateur.

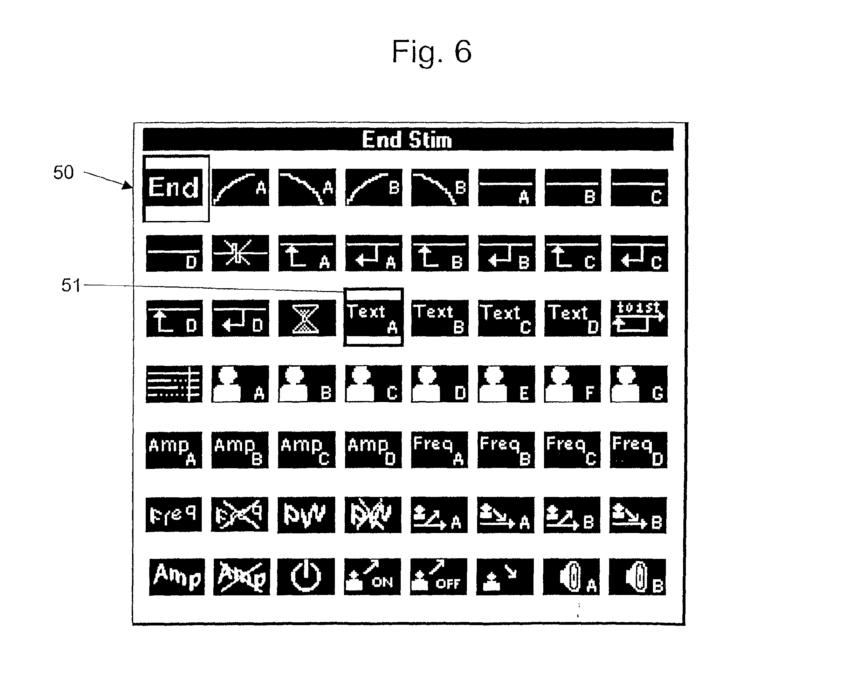

Lesdites cases des rangées peuvent être complétées successivement, depuis la première case à gauche, par des blocs unitaires de fonctions de stimulation servant à la composition arbitraire des séquences de données de stimulation. Ces blocs unitaires 51 sont tirés d'une fenêtre 50 de bibliothèque des blocs unitaires de fonctions de stimulation, vue en figure 6, qui apparaît en cliquant dans la séquence d'un canal.

En pressant sur une des touches graphiques 49 de chaque séquence, il est possible d'insérer successivement des blocs unitaires sélectionnés 51 dans des cases vides 42 de chaque séquence pour leur composition. Les touches 49 permettent également de supprimer un bloc unitaire introduit dans une position déterminée ou de supprimer le dernier des blocs de la séquence. Toute séquence de données de stimulation doit se terminer par le bloc unitaire de fin de séquence (END). Par défaut, les cases vides 42 sont préférablement complétées par cette icône de fin de séquence.

Les blocs unitaires de fonctions de stimulation faisant partie d'une bibliothèque de blocs unitaires sont représentés chacun par une icône 51 représentative de la fonction d'un bloc unitaire dans une fenêtre de bibliothèque 50 de blocs unitaires représentée à la figure 6. Dans cette fenêtre de bibliothèque, au moins 56 icônes représentent tous les blocs unitaires de fonctions de stimulation. Toutefois, d'autres icônes pourraient encore compléter la bibliothèque.

Les icônes permettent de reconnaître immédiatement quelle catégorie de fonction se cache dans tel ou tel bloc unitaire de fonctions. Un code de couleur de fond des icônes peut encore être adopté pour définir si un bloc unitaire est adapté pour tous les canaux de la même façon ou s'il peut être adapté pour chaque canal différemment. Il peut être envisagé d'employer la couleur de fond vert pour les blocs unitaires qui sont adaptés de la même façon pour tous les canaux, et la couleur de fond bleu pour les blocs unitaires qui sont adaptés différemment d'un canal à l'autre.

Lorsque la fenêtre de bibliothèque 50 apparaît sur l'écran, les icônes peuvent être copiées automatiquement dans des cases vides 42 des rangées 41 de la fenêtre principale 40 par une méthode bien connue de déplacement et de tombée ("drag and drop method" en terminologie anglaise) de l'interface utilisateur graphique ou par pression sur une touche d'insertion 49 expliquée ci-dessus. Ainsi, les séquences de données de stimulation sont composées rapidement en copiant les icônes souhaitées tirées de la fenêtre de bibliothèque. De plus comme chaque icône 51 définit visuellement la fonction du bloc unitaire, il est facile et intuitif de les placer successivement dans chaque rangée pour établir des séquences complexes de données de stimulation personnelles souhaitées.

Les blocs unitaires de fonctions de stimulation placés dans les rangées 41 pour établir des séquences de données de stimulation sont configurés dans la fenêtre principale 40. Des positions 48 dans chaque case 42 des rangées 41 permettent d'introduire par exemple des valeurs pour configurer certains blocs unitaires 51. Chaque bloc configuré est défini par au moins un numéro d'identification spécifique, qui est compris par exemple entre 0 et 255, lors de la traduction des séquences de données de stimulation, afin que ledit bloc occupe une position mémoire déterminée. Cette position mémoire est fonction également du numéro de canal de stimulation lors de l'enregistrement des séquences codées sur la carte à puce.

Des éléments ou boutons graphiques 43 au nombre de 6, montrés en bas sur la partie gauche de la fenêtre principale 40, peuvent être pressés à l'aide d'un pointeur d'une souris d'ordinateur afin d'ouvrir une fenêtre d'adaptation correspondante des paramètres à adapter. Les fenêtres d'adaptation de paramètres concernent par exemple des informations de l'utilisateur, des rampes et des constantes de largeur d'impulsions, des constantes d'amplitude et de fréquence, des commandes analogique d'amplitude en fonction de mesures de capteurs, des interactions d'utilisateur et des données textuelles.

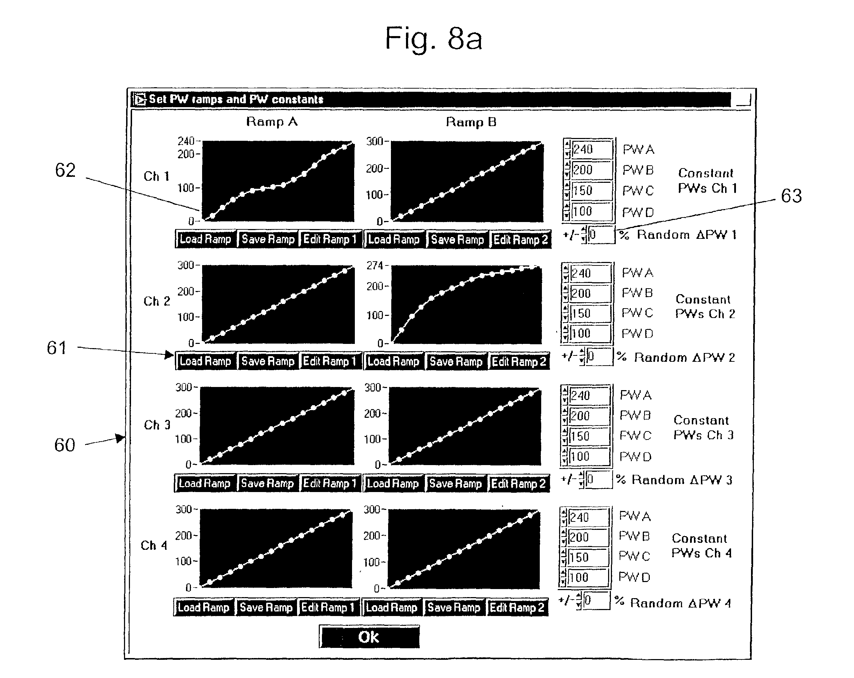

Les fenêtres d'adaptation des paramètres pour les blocs unitaires seront expliquées en détail dans la suite de la description suivant les figures 8a, 8b, 9a, 10a, 10b, 11 et 12. Par contre, la fenêtre d'informations de l'utilisateur sélectionnée par le premier bouton graphique 43 n'est pas représentée sur une figure.

Des boutons graphiques 44 de la fenêtre principale 40 peuvent être déplacés graphiquement par le pointeur pour définir le mode de stimulation. Il est possible de choisir la forme des impulsions électriques, c'est-à-dire monopolaires ou bipolaires, asymétriques ou symétriques, alternées ou non alternées. La composition des séquences de données de stimulation peut se faire en mode basé sur le temps ou en mode basé sur les impulsions. De préférence, il est choisi le mode basé sur le temps. De plus, comme plusieurs cartes à puces peuvent être programmées pour une utilisation de plusieurs appareils de stimulation interconnectés, un mode maître ou un mode esclave peut être choisi. Le mode maître ou esclave permet de définir la priorité des données à mémoriser sur l'une ou l'autre carte à puce à programmer. Bien entendu, chaque durée sélectionnée ou introduite dans la fenêtre principale est traduite dans les moyens à microcontrôleurs pour la transmission.

Des positions 45 d'introduction de valeurs par défaut ou de valeurs maximales sont prévues dans la fenêtre principale. Dans chaque rangée, il est possible d'introduire une valeur d'amplitude par défaut et des valeurs maximales de largeur d'impulsions et d'amplitude. Une valeur par défaut de fréquence de stimulation peut être également introduite pour tous les canaux de stimulation. Dans chaque rangée, il peut encore être introduit des textes pour spécifier à quel type de muscles s'applique la séquence de données de stimulation composée.

Une fois que les séquences de données de stimulation sont terminées et que les paramètres ont été adaptés pour l'exécution des blocs unitaires des séquences, il s'agit de traduire les séquences de données de stimulation en des séquences codées. Après cette étape, un bouton graphique 46 de programmation de la carte à puce est pressé pour l'enregistrement des séquences codées et des paramètres adaptés aux positions déterminées de la mémoire de la carte. La transmission des données entre la station d'ordinateur et l'appareil de stimulation se fait de préférence par voie sérielle.

Pour la programmation de la carte, une boîte de dialogue, non représentée, apparaît en pressant sur un des boutons 46. Cette boîte stipule la mise en marche de l'appareil de stimulation pour l'enregistrement des séquences codées sur la carte à puce, la connexion du câble de transfert des données, et la vérification qu'une carte a été introduite dans le dispositif de lecture et d'écriture de l'appareil.

Une boîte d'erreur, non représentée, apparaît également pour la transmission des données de stimulation. Cette boîte indique si la communication avec l'appareil de stimulation a échoué, dans le cas où l'appareil était éteint ou le câble n'était pas connecté ou aucune carte n'était introduite dans l'appareil. De plus, une boîte d'information, non représentée, montre l'avance de la procédure de déchargement du programme et indique que la carte à puce a été programmée avec succès.

Dans une étape préliminaire, un des boutons 46 permet de commander la lecture d'une carte préprogrammée insérée dans l'appareil de stimulation mis en marche. Cela permet notamment de visionner sur l'écran de la station d'ordinateur les données de la carte préprogrammée afin de pouvoir les modifier par exemple.

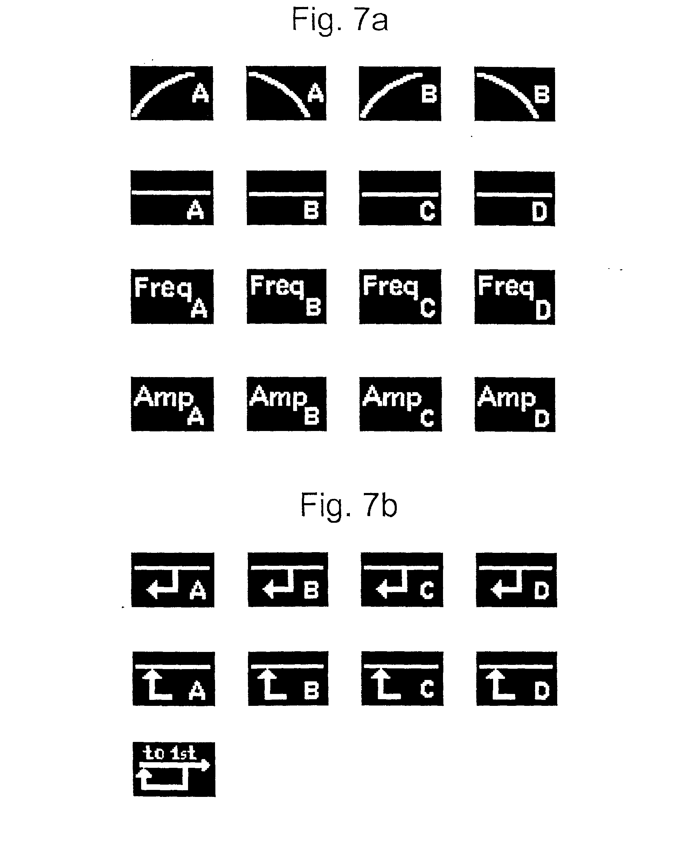

Les blocs unitaires de fonctions de stimulation sont répartis dans cinq catégories qui sont expliqués en référence aux figures 7a à 7e montrant les icônes correspondantes desdits blocs. Ces catégories concernent les blocs de stimulation, les blocs de boucles, les blocs à buts généraux, les blocs à buts spéciaux, et les blocs d'interaction de l'utilisateur avec les blocs de branchement dans les séquences.

A la figure 7a, la première rangée de blocs unitaires concernent des rampes ascendantes et descendantes A et B de largeur d'impulsions qu'il est possible d'adapter dans une fenêtre d'adaptation de rampes 60 vue à la figure 8a. Ladite fenêtre d'adaptation des rampes 60 est ouverte en sélectionnant le second bouton graphique 43 de la fenêtre principale vue aux figures 5a et 5b.

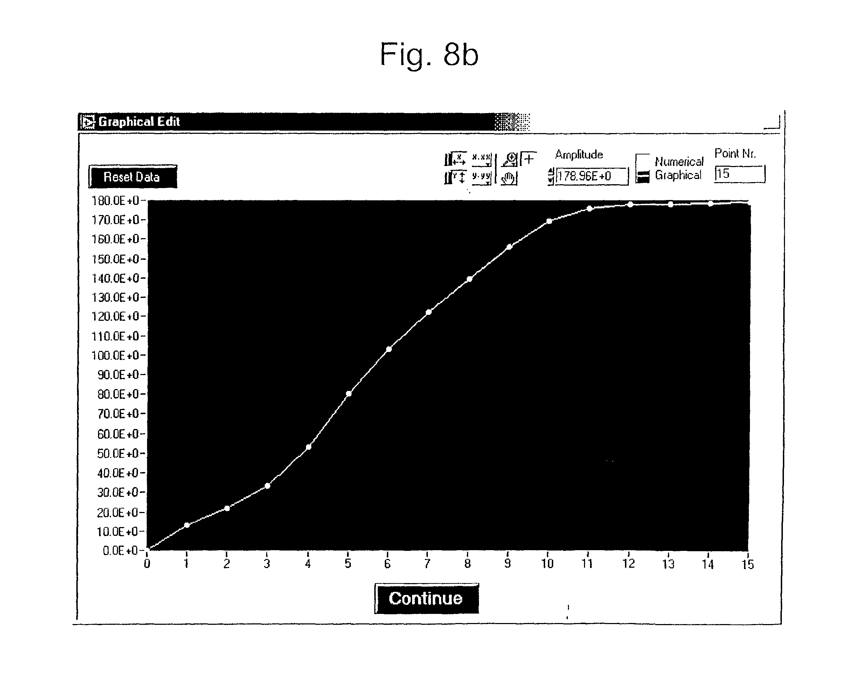

On remarque sur cette fenêtre d'adaptation des rampes 60 plusieurs graphiques 62 pour adapter les deux rampes pour chaque canal de stimulation. Par défaut, la courbe de chaque rampe est linéaire. Par contre, les courbes de la rampe A du canal 1 et de la rampe B du canal 2 ont été modifiées. Cette modification peut être réalisée dans une fenêtre d'édition, vue à la figure 8b, pour l'une ou l'autre des rampes de chaque canal en pressant sur un bouton graphique 61 d'édition des rampes.

Uniquement deux courbes de rampes A et B peuvent être dessinées par séquence de données de stimulation. Ces courbes permettent de définir deux rampes équivalentes ascendante et descendante A et deux rampes équivalentes ascendante et descendante B par canal. La figure 8b montre justement une fenêtre d'édition d'une rampe ascendante dans laquelle 16 points sont dessinés ou introduits par des moyens d'introduction de données. Pour avoir une rampe descendante basée sur la rampe ascendante, les points de la rampe descendante sont parcourus dans le temps en sens inverse de la rampe ascendante. La valeur des largeurs d'impulsions peut être définie jusqu'à 16 ms par pas de 1 µs.

Au lieu de devoir dessiner les rampes A et B de chaque canal, celles-ci peuvent aussi être chargées de fichiers de texte externes et sauvegardées en pressant des boutons de chargement de rampes et de sauvegarde 61 vus à la figure 8a.

En plus de l'adaptation de ces rampes, le bloc unitaire représentant l'une ou l'autre des rampes A et B peut être configuré dans la fenêtre principale. La durée de la rampe A ou B peut être sélectionnée entre 0,1 et 3,2 s par pas de 0,1 s. Pour générer une telle rampe, 16 différents points décrivent la rampe et son profil comme expliqué ci-dessus.

Pour obtenir une rampe, dont la longueur est 1,6 s, tous les 16 points sont utilisés dans un ordre consécutif. Par contre, pour une durée sélectionnée plus courte que 1,6 s, le programme saute quelques uns des points introduits. Par exemple, si la durée sélectionnée est 1 s, les points 2, 3, 5, 6, 8, 10, 11, 13, 14 et 16 de la courbe sont exécutés.

Dans le cas où la rampe est entre 1,7 et 3,2 s, le programme répète quelques points deux fois. Les points, qui se répètent deux fois, sont arithmétiquement distribués de manière similaire à ce que le programme exécute dans la marge de 0,1 à 1,6 s. Au lieu d'omettre des valeurs, certaines de ces valeurs sont donc répétées deux fois. Par exemple pour une durée sélectionnée de 2,2 s, les points 1, 1, 2, 3, 4, 4, 5, 6, 7, 7, 8, 9, 9, 10, 11, 12, 12, 13, 14, 15, 15 et 16 de la courbe sont exécutés.

Pour la rampe ascendante A, les numéros d'identification de 0 à 31 pour chaque canal sont attribués en fonction du nombre de points sélectionnés lors de la configuration du bloc unitaire correspondant. Pour la rampe descendante, ce sont les numéros d'identification 32 à 63 qui sont attribués. Pour la rampe ascendante B, ce sont les numéros d'identification 64 à 95 qui sont attribués. Pour la rampe descendante B, ce sont les numéros d'identification 96 à 127 qui sont attribués. Il est bien clair que le numéro d'identification attribué à un des blocs unitaires de rampe est placé dans un registre mémoire de la carte à puce relatif également au numéro du canal en question.

L'attribution des numéros d'identification aux bloc unitaires de rampes de largeur d'impulsions permet d'offrir une large variété de configurations possibles de rampes de largeur d'impulsions sur la base d'une rampe ascendante ou descendante pour chaque canal de stimulation. Comme les séquences sont définies avec les numéros d'identification des blocs configurés à enregistrer sur la carte, cela permet de réduire l'espace mémoire nécessaire pour les séquences codées complexes.

A la figure 7a, la seconde rangée de blocs unitaires concerne des constantes A, B, C et D de largeur d'impulsions au nombre de quatre par canal. Les valeurs des constantes de largeur d'impulsions de chaque canal sont introduites dans des positions déterminées à côté des références PWA, PWB, PWC et PWD de la fenêtre d'adaptation visible à la figure 8a. Ces valeurs de constantes peuvent donc être différentes d'un canal à l'autre.

Chaque bloc unitaire de constantes de largeur d'impulsions peut être configuré dans la fenêtre principale 40, visible en figures 5a et 5b, en introduisant des valeurs temporelles (dans un mode basé sur le temps) allant de 0,1 s à 25,5 s. En fixant une valeur temporelle, on impose que les impulsions de courant doivent avoir une largeur d'impulsions fonction de la constante fixée pendant la durée spécifiée.

Dans ce cas de figure, plusieurs numéros d'identification peuvent définir la configuration de chaque bloc de constantes par addition des durées spécifiées de chaque numéro d'identification (addition binaire). Chaque numéro d'identification définit une durée qui est un multiple de deux ou une division par deux de la durée du numéro d'identification voisin. Les durées des numéros sont 0,1 s, 0,2 s, 0,4 s, 0,8 s, 1,6 s, 3,2 s, 6,4 s et 12,8 s ce qui permet par addition de l'une ou de l'autre de ces durées de couvrir toutes les durées de 0,1 s à 25,5 s.

Pour les constantes A, B, C et D du premier canal, les numéros d'identification de 128 à 135 sont attribués pour les valeurs de durée spécifiées ci-dessus. Pour les constantes A, B, C et D du second canal, les numéros d'identification de 136 à 143 sont attribués. Pour les constantes A, B, C et D du troisième canal, les numéros d'identification de 144 à 151 sont attribués. Pour les constantes A, B, C et D du quatrième canal, les numéros d'identification de 152 à 159 sont attribués. Bien entendu, pour chaque canal, chaque constante est mémorisée dans des registres mémoires déterminés de la carte à puce en fonction des numéros d'identification nécessaires pour la durée de configuration introduite.

A la figure 7a, la troisième rangée de blocs unitaires de fonctions de stimulation concerne la fréquence des impulsions de stimulation. Quatre valeurs de fréquences A, B, C et D peuvent être introduites pour tous les canaux dans des emplacements 71 d'une fenêtre d'adaptation de fréquence et d'amplitude 70 vue à la figure 9a. Ladite fenêtre d'adaptation de fréquence et d'amplitude est ouverte en sélectionnant un troisième bouton graphique 43 de la fenêtre principale 40 vue aux figures 5a et 5b.

Il est à noter que la fréquence doit être la même pour tous les canaux de stimulation, ce qui fait que si un bloc unitaire de fréquence est introduit dans une séquence, elle est appliquée également pour tous les autres canaux de stimulation. La valeur de fréquence peut être choisie entre 0 et 100 Hz par pas de 1 Hz.

Pour les quatre valeurs de fréquences A, B, C et D introduites, les numéros d'identification de 220 à 223 sont attribués à chaque bloc unitaire de fréquence.

A la figure 7a, la quatrième rangée de blocs unitaires de fonctions de stimulation concerne des constantes d'amplitude à atteindre. Quatre valeurs d'amplitudes A, B, C et D à atteindre peuvent être introduites différemment pour chaque canal dans des emplacements 73 de la fenêtre d'adaptation de fréquence et d'amplitude 70 vue à la figure 9a. En plus des valeurs d'amplitudes introduites, il est nécessaire de définir le temps de transition de chaque valeur d'amplitude pour le passage d'une valeur d'amplitude initiale à la valeur introduite correspondante dans cette fenêtre. Selon les besoins, quatre jeux de valeurs d'amplitude différents chacun avec un temps de transition déterminé sont introduits pour les quatre canaux de stimulation.

Pour les quatre valeurs d'amplitudes A, B, C et D introduites, les numéros d'identification de 216 à 219 sont attribués à chaque bloc unitaire d'amplitude. En plus des numéros d'identification des blocs unitaires d'amplitude utilisés pour les séquences codées, les données concernant le numéro du canal devra être également envoyé afin d'enregistrer les numéros d'identification dans des positions mémoires déterminées relatives au numéro du canal de stimulation.

A la figure 7b, des blocs unitaires pour définir des boucles de répétition de parties de séquence différemment pour chaque canal de stimulation sont montrés. Quatre sortes de boucles A, B, C et D de répétition à configurer peuvent être placées pour chaque séquence de données de stimulation. De plus, une boucle définie par une seule icône est montrée dans la troisième rangée pour opérer une répétition depuis le début de la séquence de données. Le numéro d'identification 237 est attribué au bloc de répétition depuis le début de la séquence.

Deux blocs unitaires pour chaque boucle sont nécessaires pour donner l'endroit de départ d'une boucle et l'endroit d'arrivée de la boucle correspondante. La première rangée de la figure 7b montre les blocs unitaires de départ des boucles A, B, C et D alors que la deuxième rangée montre les blocs unitaires d'arrivée ou de marquage des boucles A, B, C et D.

Les blocs unitaires A, B, C et D de la première rangée peuvent être configurés pour définir le nombre de répétition de parties de séquence désiré. Il est bien clair que pour répéter une partie d'une séquence de données de stimulation ces blocs unitaires de départ sont placés en des positions de séquence qui ne doivent pas précéder les blocs unitaires d'arrivée correspondant.

A titre illustratif, la figure 5a montre par exemple dans la séquence du premier canal de stimulation une boucle de répétition grâce au bloc unitaire de départ A placé dans la huitième case et au bloc unitaire d'arrivée A placé dans la deuxième case de la séquence. Le bloc unitaire de départ A est configuré de telle sorte que la partie de séquence insérée entre les deux blocs de boucle doit se répéter 7 fois.

Le nombre de répétitions d'une boucle peut être choisi entre 1 fois à 255 fois. Pour obtenir toutes ces valeurs de répétition, plusieurs numéros d'identification, représentant chacun un nombre déterminé de répétitions, peuvent définir la configuration de chaque bloc unitaire de départ de boucle dans chaque séquence. Les nombres déterminés de répétition des numéros d'identification sont 1 fois, 2 fois, 4 fois, 8 fois, 16 fois, 32 fois, 64 fois et 128 fois, ce qui permet par addition de l'un ou de l'autre de ces nombres déterminés de couvrir le nombre de répétitions d'une boucle de 1 fois à 255 fois.

Les numéros d'identification de 169 à 176 du bloc unitaire de départ A sont attribués pour les nombres déterminés de répétition spécifiés ci-dessus, alors que le numéro d'identification 168 est attribué pour le bloc unitaire d'arrivée A. Les numéros d'identification de 178 à 185 du bloc unitaire de départ B sont attribués pour les nombres déterminés de répétition spécifiés ci-dessus, alors que le numéro d'identification 177 est attribué pour le bloc unitaire d'arrivée B. Les numéros d'identification de 187 à 194 du bloc unitaire de départ C sont attribués pour les nombres déterminés de répétition spécifiés ci-dessus, alors que le numéro d'identification 186 est attribué pour le bloc unitaire d'arrivée C. Les numéros d'identification de 196 à 203 du bloc unitaire de départ D sont attribués pour les nombres déterminés de répétition spécifiés ci-dessus, alors que le numéro d'identification 195 est attribué pour le bloc unitaire d'arrivée D.

Lors de la configuration des blocs de départ de boucles, il peut être également choisi la valeur 0 pour définir que la boucle se répète indéfiniment.

Comme les numéros d'identification définissant les boucles de répétitions peuvent être les mêmes pour plusieurs canaux, une indication du numéro du canal doit être transmis avec les séquences codées pour que chaque boucle soit mémorisée dans des registres mémoires déterminés de la carte à puce. Les positions mémoires, pour chaque canal, sont fonction des numéros d'identification nécessaires pour obtenir le nombre souhaité de répétitions de boucle.

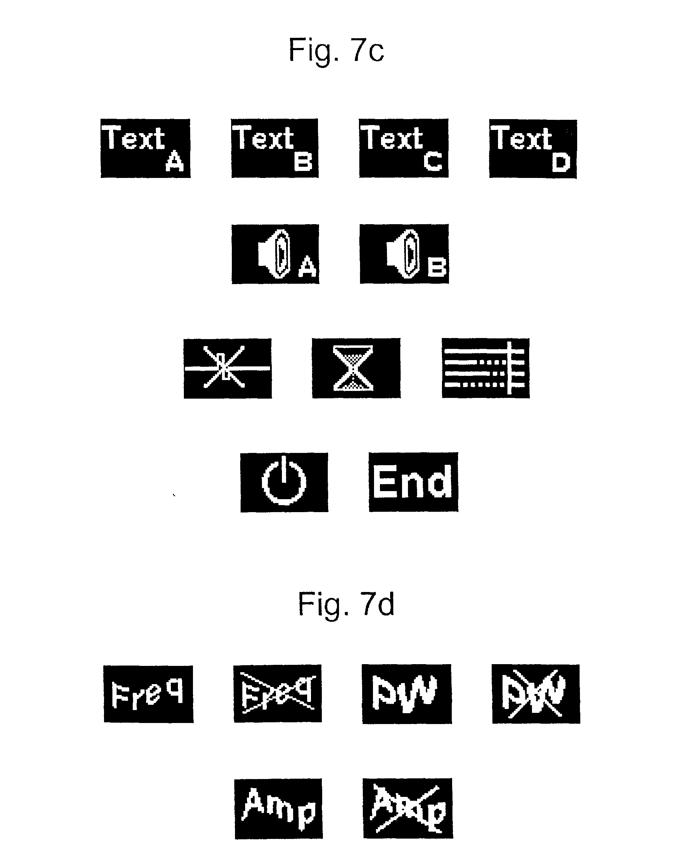

A la figure 7c, différents blocs unitaires à buts généraux sont représentés. La première rangée concerne quatre blocs unitaires A, B, C et D de texte utilisés pour afficher au maximum deux lignes de texte sur l'écran d'un appareil de stimulation électrique pendant l'exécution des séquences de stimulation de l'appareil. Ces quatre blocs de texte sont définis pour tous les canaux de la même façon.

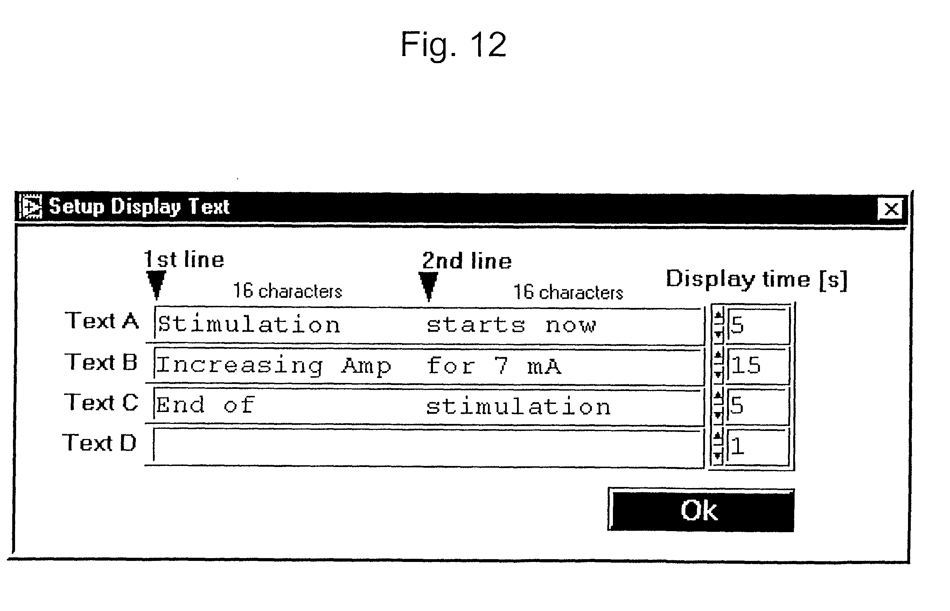

L'introduction des messages d'information des quatre blocs A, B, C et D de texte est réalisée dans une fenêtre d'adaptation de textes vue à la figure 12. Ladite fenêtre d'adaptation des textes est ouverte en sélectionnant un sixième bouton graphique 43 de la fenêtre principale 40 vue aux figures 5a et 5b. Chaque message est introduit dans des lignes de textes A, B, C et D.

Il est possible d'introduire au maximum 2 portions de message avec au maximum 16 caractères pour chaque portion ce qui correspond aux possibilités d'affichage actuelle de l'écran d'un appareil de stimulation électrique, tel que l'appareil Compex2. De plus, il peut être introduit aux extrémités de chaque ligne de message A, B, C et D des temps d'affichage sur l'écran de l'appareil de stimulation.

Les numéros d'identification 233 à 236 sont attribués pour les blocs unitaires de texte afin d'occuper des positions mémoires déterminées sur la carte à puce indépendamment du numéro du canal correspondant.

Dans la seconde rangée de la figure 7c, deux blocs unitaires A et B d'alarme sonore sont montrés. Ces deux blocs d'alarme A et B peuvent être adaptés différemment pour chaque canal de stimulation avec des tonalités ou des mélodies différentes. Cela permet d'avertir par exemple l'instant où un canal de stimulation est destiné à opérer une contraction de certains muscles à stimuler. Normalement dans le cas le plus simple, les alarmes sont ajustées à une fréquence sonore basse pour l'une et plus haute pour l'autre pour les différencier. Les numéros d'identification 239 et 240 sont attribués à ces blocs de variation aléatoire de largeur d'impulsions.

Dans la troisième rangée de la figure 7c, un premier bloc unitaire concerne une absence d'impulsions de stimulation, dans chaque séquence dans laquelle il est introduit. L'absence d'impulsions est définie par une période de temps fixée lors de la configuration dudit bloc d'absence d'impulsions. La durée d'absence d'impulsions peut être définie de 0,1 s à 25,5 s. Pour ce faire, plusieurs numéros d'identification, représentant les durées 0,1 s, 0,2 s, 0,4 s, 0,8 s, 1,6 s, 3,2 s, 6,4 s et 12,8 s, peuvent être cumulés dans une séquence codée pour atteindre la valeur de configuration souhaitée. Les numéros d'identification 160 à 167 sont attribués aux durées ci-dessus.

Le deuxième bloc unitaire de cette troisième rangée concerne un bloc unitaire de délai. Ce bloc permet de garder pendant un temps déterminé la dernière valeur de largeur d'impulsions ou d'amplitude du bloc unitaire qui précède le bloc de délai dans une séquence dans laquelle il est placé. Ce bloc de délai peut être défini par plusieurs numéros d'identification qui caractérisent 1 cycle, 2 cycles, 4 cycles, 8 cycles, 16 cycles, 32 cycles, 64 cycles et 128 cycles pour pouvoir donner par cumul 255 cycles au total (25,5 s). Les numéros d'identification de 204 à 211 sont attribués pour occuper des positions mémoires sur la carte à puce en fonction également du numéro du canal correspondant.

Finalement le troisième bloc unitaire de cette troisième rangée concerne un bloc unitaire de synchronisation qui impose une synchronisation pour tous les canaux de stimulation. Le numéro d'identification 238 est attribué à ce bloc unitaire de synchronisation.

Dans la dernière rangée de la figure 7c, des blocs unitaires d'arrêt ou de fin de séquences sont représentés. Le premier bloc unitaire placé dans n'importe quelle séquence d'un canal de stimulation permet d'arrêter ou d'interrompre l'appareil de stimulation. Le numéro d'identification 230 est attribué à ce bloc unitaire.

Le second bloc unitaire de cette dernière ligne est le bloc de fin de séquence. Il est placé comme dernier bloc unitaire dans chaque séquence de données de stimulation. Par défaut, ce bloc de fin de séquence remplit les cases vides 42 des rangées de séquence 41 de la fenêtre principale 40 vue à la figure 5a. Le numéro d'identification 255 est attribué à ce bloc de fin de séquence.

A la figure 7d, des blocs unitaires à buts spéciaux sont représentés. Les deux premiers blocs unitaires de la première ligne concernent d'une part l'introduction d'une variation aléatoire de fréquence et d'autre part l'interruption de la variation aléatoire de fréquence. La variation aléatoire de fréquence est faite de la même façon pour tous les canaux autour d'une valeur déterminée de fréquence. Le pourcentage de variation aléatoire peut être introduit dans un emplacement 72 de la fenêtre d'adaptation de fréquence et d'amplitude 70 vue à la figure 9a. Les numéros d'identification 224 et 225 sont attribués à ces blocs de variation aléatoire de fréquence.

Les deux derniers blocs unitaires de la première ligne de cette figure 7d concernent d'une part l'introduction d'une variation aléatoire de largeur d'impulsions et d'autre part l'interruption de la variation aléatoire de largeur d'impulsions. La variation de largeur d'impulsions est définie autour d'une valeur déterminée de largeur d'impulsions qui peut être différente pour chaque canal de simulation. Le pourcentage de variation aléatoire de largeur d'impulsions peut être introduit pour chaque canal dans des emplacements 63 de la fenêtre d'adaptation des rampes de largeur d'impulsions 60 vue à la figure 8a. Les numéros d'identification 226 et 227 sont attribués à ces blocs de variation aléatoire de largeur d'impulsions.

Les blocs unitaires de la seconde ligne de la figure 7d concernent d'une part l'introduction d'une variation aléatoire d'amplitude et d'autre part l'interruption de variation aléatoire d'amplitude. La variation d'amplitude est définie autour d'une valeur déterminée d'amplitude. Le pourcentage de variation d'amplitude peut être introduit pour chaque canal dans des emplacements 74 de la fenêtre d'adaptation de fréquence et d'amplitude 70 vue à la figure 9a. Les numéros d'identification 241 et 242 sont attribués à ces blocs de variation aléatoire de largeur d'impulsions.

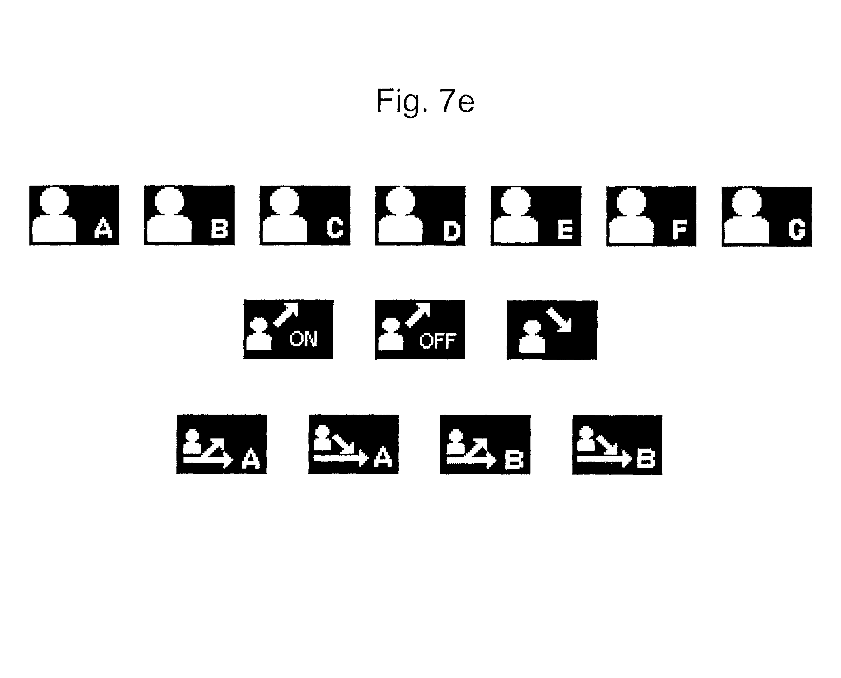

A la figure 7e, tous les blocs unitaires relatifs à des interactions d'un utilisateur sont représentés. On entend par interaction toute manipulation opérée par un utilisateur à l'aide de capteurs ou d'actionneurs pour des commandes lors du déroulement de chaque séquence de données de stimulation. Ces capteurs ou ces actionneurs sont utilisés principalement dans le domaine médical pour des neuroprothèses afin de commander par exemple les mouvements et la force ou le couple développé d'un membre handicapé ou accidenté.

Les blocs unitaires d'interaction A, B, C, D, E, F et G de la première rangée concernent des actions opérées par un utilisateur dans le déroulement de la séquence de données de stimulation dans laquelle ils peuvent être placés. Les actions sont fournies par des mesures de capteurs ou d'actionneurs qui peuvent être connectés, par exemple, à trois entrées d'un appareil de stimulation, tel que l'appareil Compex2, lors de sa mise en fonction.

Cet appareil Compex2 comprend notamment deux entrées de mesures du type analogique ou du type numérique qui utilisent les canaux A et B sur l'arrière de l'appareil. Une autre entrée désignée C à buts multiples est utilisée d'une part pour la connexion par exemple d'un bouton-poussoir de commande, et d'autre part pour le transfert sériel par un câble (RS-232) des séquences de données de stimulation codées établies dans la station d'ordinateur. Cette dernière entrée sert également pour l'alimentation électrique de l'appareil ou pour charger électriquement un accumulateur de l'appareil.

Les numéros d'identification 248 à 254 sont attribués aux blocs unitaires d'interaction A, B, C, D, E, F et G. Quant au type d'actionneur ou de capteur choisi pour chaque bloc unitaire d'interaction, cela sera expliqué ci-dessous en référence aux figures 10a et 10b.

Les blocs unitaires de la seconde rangée de la figure 7e concernent des blocs unitaires pour marquer notamment des interruptions sous l'action d'un utilisateur. Ces blocs sont configurés avec un bloc unitaire d'interaction A, B, C, D, E, F ou G pour tous les canaux de la même manière, comme représenté dans le bloc d'interruption 87 de la fenêtre d'adaptation des interactions de l'utilisateur 80 à la figure 10a. Les premier et second blocs unitaires de marquage d'interruption ON et OFF définissent d'une part le point de départ dans une séquence de l'action d'un utilisateur par exemple pour arrêter la séquence, et d'autre part la fin de l'action d'un utilisateur dans ladite séquence. Si une interaction de l'utilisateur est intervenue dans le temps entre les deux premiers blocs, un saut de séquence est fait jusqu'au troisième bloc unitaire qui définit la position d'une routine d'urgence, par exemple la fin de séquence. L'action de l'utilisateur pour l'interruption d'une séquence ne peut donc être réalisée dans le temps qu'entre les deux premiers blocs unitaires. Ces deux premiers blocs unitaires de marquage d'interruption portent les numéros d'identification 231 et 232.

La dernière rangée de la figure 7e montre deux paires de blocs unitaires de branchement A et B dans une séquence de données de stimulation sous l'action d'au moins un utilisateur. Chaque paire de blocs unitaires comprend un premier bloc de point de départ du branchement et un second bloc de l'endroit d'arrivée du branchement. Un premier bloc de départ de branchement peut être placé dans une première position d'une séquence de données de stimulation suivi plus loin d'un second bloc d'arrivée de branchement placé dans une seconde position de ladite séquence. Lors du déroulement de la séquence de données de stimulation, l'utilisateur a le choix de laisser la séquence se dérouler normalement ou de passer de la première position à la seconde position de séquence par une action de sa part.

Le bloc du point de départ de branchement A ou B est adapté ou configuré avec deux blocs unitaires différents d'interaction A, B, C, D, E, F ou G, comme représenté dans les blocs de branchement par l'utilisateur 86 de la fenêtre d'adaptation des interactions de l'utilisateur 80 à la figure 10a. Un premier bloc d'interaction permet de sauter la séquence de la première position à la seconde position, tandis que le second bloc d'interaction permet de continuer la séquence.

Les numéros d'identifications 228 et 229 sont attribués pour chaque paire de blocs de branchement ou chaque branche A et B d'interaction par l'utilisateur.

A la figure 10a, une fenêtre d'adaptation d'interactions d'un utilisateur 80 est montrée. Sept interactions utilisateurs peuvent être adaptés dans cette fenêtre 80. Cette fenêtre 80 est ouverte en pressant un cinquième bouton graphique 43 depuis la fenêtre principale 40 vue aux figures 5a et 5b.

Dans la première colonne de menus graphiques 81, relatifs chacun à un bloc unitaire d'interaction A, B, C, D, E, F et G, il peut être choisi l'entrée de branchement du capteur ou de l'actionneur sur l'appareil de stimulation électrique correspondant au bloc d'interaction sélectionné. Précédemment, il a été indiqué que ledit appareil dispose d'au moins trois entrées pour des mesures de capteurs ou d'actionneurs qui sont du type analogique ou numérique. Bien entendu, il peut être choisi également dans la colonne 81 de ne pas sélectionner d'entrée de l'appareil.

Dans la seconde colonne de menus graphiques 82, relatifs chacun à un bloc unitaire d'interaction A, B, C, D, E, F et G, il est spécifié le type de capteur ou d'actionneur utilisé. Il peut être choisi par exemple un actionneur à commande par le pied, qui comprend des résistances sensibles à la force, un gyroscope, un bouton-poussoir, un bouton du clavier de l'appareil de stimulation, un capteur électromyographique (EMG).

Dans la troisième et la quatrième colonnes de boutons graphiques 83 et 84, relatifs chacun à un bloc unitaire d'interaction A, B, C, D, E, F et G, des critères d'adaptation relatifs au type de capteur ou d'actionneur peuvent être introduits pour fixer des niveaux de mesure pour l'interaction de l'utilisateur. En pressant sur un bouton 84 SET, par exemple, une fenêtre de critères de déclenchement, montrée à la figure 10b, apparaît notamment pour définir la courbe attendue pour continuer la stimulation dans le temps avec un capteur électromyographique ou d'autres capteurs. Des courts messages d'information peuvent être également introduits dans des emplacements 85 de la fenêtre 80. Au cas où le critère de déclenchement est chargé ou sauvegardé, c'est le nom du fichier qui apparaîtra dans la petite fenêtre.

Lorsqu'un capteur fournit une tension de mesure basée sur la force ou le couple qui lui est appliqué, une table d'établissement d'amplitudes des impulsions générées peut être adaptée pour chaque canal différemment. La figure 11 montre une fenêtre de la table d'établissement d'amplitudes 90 qui est ouverte en sélectionnant un quatrième bouton graphique 43 depuis la fenêtre principale 40 vue aux figures 5a et 5b. Cette fenêtre 90 montre quatre graphiques 91 des quatre canaux de stimulation pour imposer une amplitude aux impulsions électriques sur la base de la mesure en tension de capteurs. L'amplitude du signal d'entrée du capteur sur l'axe des x entre 0 et 5V est adaptée à l'amplitude des impulsions de stimulation sur l'axe des y. Lesdits capteurs sont utilisés pour l'interaction volontaire d'un utilisateur ou pour l'interaction due à la mesure de la réponse musculaire suite à la stimulation électrique.

Des boutons graphiques 92 de chaque canal peuvent être sélectionnés pour éditer une rampe d'amplitude d'impulsions pour adapter le tracé des signaux de capteurs, pour sauvegarder la rampe introduite ou pour charger une rampe, qui peut provenir d'un fichier de texte externe. Des menus graphiques 93 permettent d'attribuer un type d'entrée de l'appareil de stimulation pour chaque canal de stimulation.

Dans l'emploi d'appareil de stimulation électrique en tant que prothèse, il est avantageux que son utilisateur puisse le commander par des parties du corps valides. Dans le cas d'une prothèse de la main, l'autre main ou bras peut servir à commander ladite prothèse à l'aide de capteurs fournissant des réponses électriques dépendant de l'action musculaire volontaire du bras ou de la main valide. Ceci permet d'imposer des mouvements à la main handicapée par génération de stimulation électrique.

Pour l'actionnement d'une main, trois canaux de stimulation sont nécessaires sur lesquels une paire d'électrodes de surface par canal est placée aux endroits spécifiques pour l'action de la main. Le patient peut réguler la façon dans laquelle le pouce fléchit et l'intensité de la force de saisie afin de lui permettre d'ajuster la saisie à la taille et la forme de l'objet qu'il souhaite prendre. Dans le cas où le muscle triceps du bras doit être activé électriquement, un quatrième canal doit être utilisé. La commande des stimulations électriques pour la saisie de l'objet peut être opérée par un bouton-poussoir.

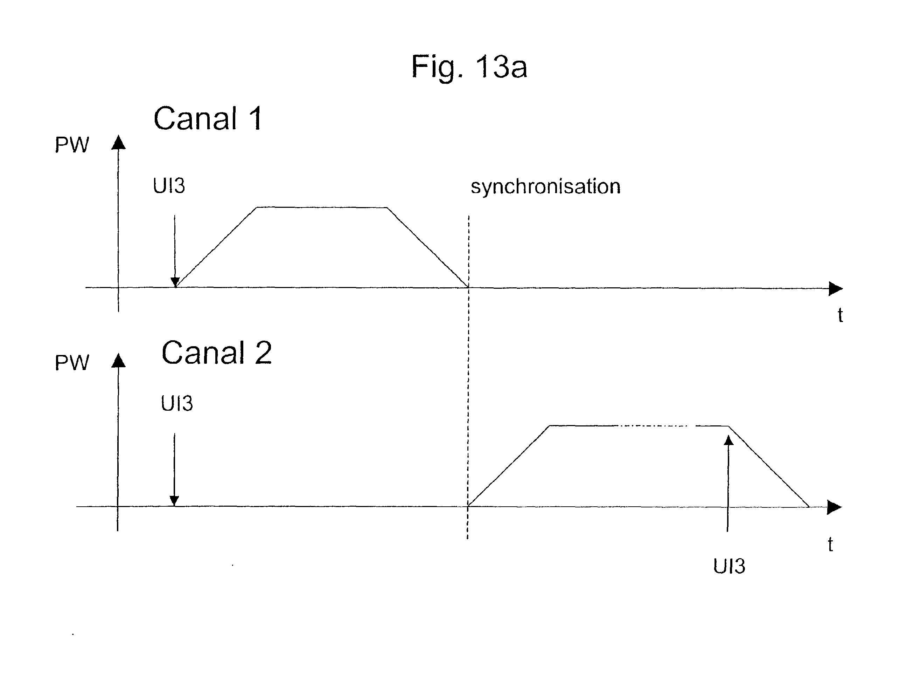

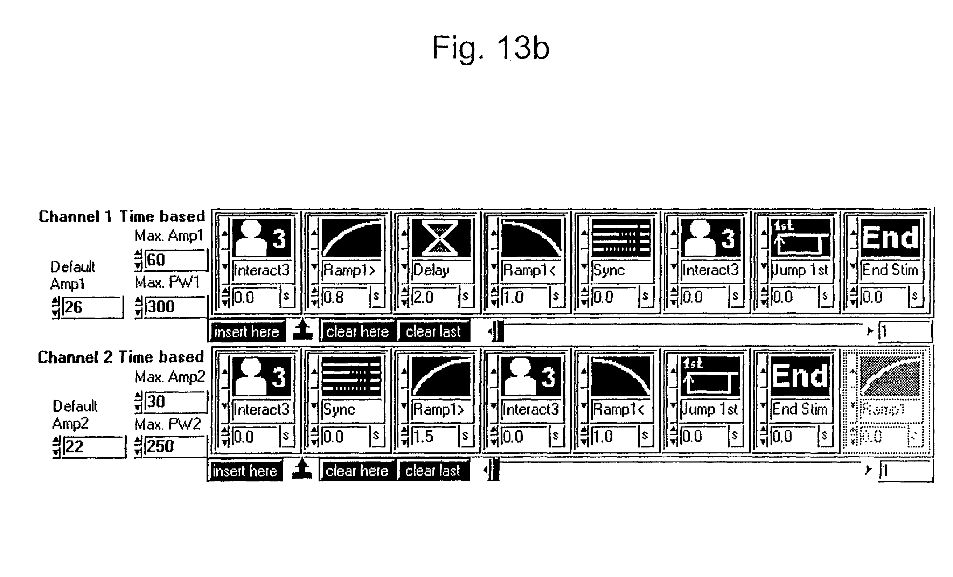

A titre illustratif, un exemple de programmation de séquences de données de stimulation dans la fenêtre principale pour deux canaux de stimulation et les graphiques de largeur d'impulsions PW correspondant sont représentés aux figures 13a et 13b. La génération des impulsions électriques, avec la variation de largeur d'impulsions des deux canaux, débute et se termine sur la base d'une interaction d'un utilisateur UI3, en fonction du temps. Un bloc unitaire de synchronisation entre les deux canaux impose que le second canal débute ces rampes de largeur d'impulsions à la fin des rampes de largeur d'impulsions du premier canal.

Dans ces figures 13a et 13b, les deux séquences de données de stimulation pour les deux canaux de stimulation sont établies pour permettre à une neuroprothèse placée au niveau d'une main d'un utilisateur de réaliser une ouverture de doigts et une fermeture de doigts. L'ouverture et la fermeture des doigts sont faites grâce à la commande d'un bouton-poussoir actionné par l'autre main de l'utilisateur.

En ce qui concerne les capteurs électromyographiques pour une commande analogique EMG, le sujet peut volontairement, graduellement et sélectivement contracter les branches antérieures et postérieures d'un muscle sur lequel il est placé pour pouvoir réguler l'activité EMG. Par exemple, il peut être placé deux capteurs EMG sur les deux branches du bras opposé à celui portant une neuroprothèse. En maintenant la branche antérieure ou postérieure contractée, il impose aux muscles de la main du bras opposé de garder la position escomptée. L'amplitude de la différence entre les signaux EMG de la branche antérieure et postérieure est utilisée pour commander la force de saisie de la main.

Une commande digitale EMG peut être utilisée par des patients qui ne peuvent réguler les signaux EMG d'une manière graduelle ou ne peuvent pas maintenir la contraction musculaire pendant des périodes prolongées. Des commandes ON et OFF sont générées dans ce cas de figure pour permettre à la prothèse de générer des séquences de stimulation appropriées.
En lieu et place des capteurs EMG, un patient peut utiliser également des boutons-poussoirs, comme décrit ci-dessus, pour activer la neuroprothèse. Le temps d'ouverture ou de fermeture de la main avec la neuroprothèse peut être fixée au préalable à 2 s.
Un principe équivalent peut être utilisé pour des personnes qui ont par exemple une bonne commande d'une jambe, mais dont l'autre nécessite l'aide d'une prothèse à stimulation électrique. Ces sujets sont typiquement des utilisateurs de chaises roulantes ou des personnes qui ne peuvent fléchir ou étendre l'articulation de la cheville et qui ont des commandes affaiblies à la hanche ou dans l'articulation des genoux. Par contre, les patients doivent avoir un bon sens d'équilibre et pouvoir rester debout sans danger en utilisant des béquilles ou des supports pour marcheurs. Une telle neuroprothèse permet ainsi d'améliorer les performances de marche en générant des séquences de la démarche dans la jambe abîmée.

Les prothèses à stimulation électrique peuvent être utilisées de manière permanente ou pour la réhabilitation d'un membre meurtri des suites d'un accident, par exemple. Ainsi, les patients qui débutent leur entraînement tôt après leur accident pourraient potentiellement bénéficier d'effets positifs de l'entraînement avec l'appareil de stimulation électrique fonctionnelle à électrodes de surface. Une programmation des séquences de données de stimulation sur une carte à puce dans ce cas est réalisée rapidement à l'aide de l'installation de programmation.

A partir de la description ci-dessus relative essentiellement à la programmation de séquences de données de stimulation sur une carte à puce pour une utilisation dans une neuroprothèse, de multiples variantes de réalisation du procédé de programmation ou de l'installation de programmation peuvent être faites sans sortir du cadre de l'invention à la connaissance de l'homme du métier. Par exemple, le support de mémorisation peut être une disquette souple ou un disque compact ou un élément de mémorisation amovible autre qu'une carte à puce décrite. Ledit support de mémorisation pourrait également être un élément de la station d'ordinateur.

Le nombre de canaux de stimulation pour l'établissement des séquences de données de stimulation sur la station d'ordinateur peut être différent des quatre canaux décrits. De plus, la fenêtre principale, où les séquences de stimulation sont composées, pourraient selon les applications comprendre des images du membre sur lequel doit être placé une neuroprothèse. La vision du membre dans la fenêtre principale permettrait de faciliter la réalisation des séquences de stimulation par une personne non spécialisée dans ce domaine technique tout en garantissant que des valeurs limites d'amplitude, de fréquence ou de largeur d'impulsions ne puissent pas être dépassées par mesure de sécurité.

## Revendications

1. Procédé de programmation de données de stimulation électrique pour un appareil de stimulation électrique neuromusculaire ou fonctionnelle (2) dans une installation qui comprend une station d'ordinateur (1) à programme de gestion de données de stimulation électrique et à interface utilisateur graphique (4), au moins un dispositif de lecture et d'écriture (18) en communication avec la station d'ordinateur (1), et au moins un support de mémorisation (8) placé dans le dispositif (18) pour mettre en mémoire des données personnelles de stimulation électrique, le procédé se **caractérise en ce qu'**il comprend les étapes de :
- composer une séquence de données de stimulation électrique (41) avec des blocs unitaires de fonctions de stimulation pour au moins un canal de stimulation d'un appareil de stimulation électrique neuromusculaire ou fonctionnelle (2) en plaçant successivement dans une fenêtre principale (40) de l'interface utilisateur graphique de la station d'ordinateur des icônes (51) représentatives chacune de la fonction d'un bloc unitaire correspondant, les icônes étant tirées d'une fenêtre de bibliothèque de blocs unitaires de fonctions de stimulation (50), et des données de configuration étant appliquées à certains blocs placés dans la séquence,
- traduire dans une unité centrale de traitement (10) de la station d'ordinateur (1) la séquence de données de stimulation composée d'une série de blocs unitaires de fonctions de stimulation configurés en une séquence de données de stimulation codée, dans laquelle chaque bloc unitaire de fonctions configuré est défini par au moins un numéro d'identification spécifique,
- transférer la séquence de données de stimulation codée à destination du dispositif de lecture et d'écriture (18), et
- commander l'enregistrement de la séquence de données de stimulation codée sur le support de mémorisation (8) placé dans ledit dispositif de lecture et d'écriture.

2. Procédé selon la revendication 1, **caractérisé en ce que** plusieurs séquences de données de stimulation électrique (41), pour respectivement plusieurs canaux de stimulation d'un appareil de stimulation électrique fonctionnelle (2), sont composées dans la fenêtre principale (40) de l'interface utilisateur graphique de la station d'ordinateur (1) à l'aide d'icônes (51) tirées de la fenêtre de bibliothèque des blocs unitaires (50), et **en ce que** les séquences de données de stimulation sont traduites en des séquences de données de stimulation codées qui sont placées les unes après les autres pour leur transfert à destination du dispositif de lecture et d'écriture (18) et leur enregistrement sur le support de mémorisation (8).

3. Procédé selon la revendication 2, **caractérisé en ce que** la composition des séquences de données de stimulation dans la fenêtre principale (40) se fait en plaçant sur un nombre de rangées (41) correspondant au nombre de canaux de stimulation les icônes (51) de construction de chaque séquence, chaque séquence des rangées étant représentée dans la fenêtre principale par un certain nombre de cases (42) visibles successives, dont chaque case peut recevoir une icône tirée de la fenêtre de bibliothèque de blocs unitaires (50), un curseur graphique (47) par rangée permettant de visionner la totalité des icônes placées de chaque séquence composée.

4. Procédé selon l'une des revendications 1 et 2, **caractérisé en ce que** la ou les séquences de données de stimulation codées sont enregistrées sur ledit support de mémorisation, qui est une carte à puce (8), en des positions mémoire déterminées correspondant aux numéros d'identification des blocs unitaires de fonctions de stimulation configurés des séquences, ainsi qu'au numéro du canal de stimulation respectif de chaque séquence.

5. Procédé selon la revendication 1, **caractérisé en ce que** les blocs unitaires de fonctions de stimulation définissent notamment des rampes d'amplitude des impulsions électriques, des rampes de largeur d'impulsions, des constantes de largeur d'impulsions, des constantes de fréquence des impulsions pour tous les canaux utilisés, des données de variation aléatoire d'amplitude d'impulsions, des données de variation aléatoire de largeur d'impulsions, des données de variation aléatoire de fréquence d'impulsions, des opérations de bouclage, des branchements dans une séquence, des données textuelles, des données sonores, des interactions d'un utilisateur, des pauses, des périodes sans impulsions électriques, une synchronisation entre canaux, des interruptions de l'appareil de stimulation, des fins de séquence.

6. Procédé selon l'une des revendications 1 et 2, **caractérisé en ce que** des paramètres pour l'exécution de blocs unitaires de fonctions de stimulation placés dans la ou les séquences de données de stimulation sont adaptés en cliquant sur au moins un élément graphique (43) caractéristique des paramètres à adapter dans la fenêtre principale (40) de l'interface utilisateur graphique pour faire apparaître au moins une fenêtre d'adaptation de paramètres (60, 70, 80, 90), **en ce que** les données relatives aux paramètres adaptés sont transmis avec la ou les séquences de données de stimulation codées à destination du dispositif de lecture et d'écriture (18), et **en ce que** les données relatives aux paramètres adaptés sont enregistrés sur le support amovible de mémorisation (8) en des positions déterminées.

7. Procédé selon la revendication 6, **caractérisé en ce que**, dans au moins une fenêtre d'adaptation de paramètres (60, 70, 80, 90), des données de paramètres sont introduits ou sélectionnés pour chaque canal de stimulation en des positions déterminées de la fenêtre d'adaptation correspondante, les données de paramètres étant des valeurs d'amplitude, de fréquence ou de largeur d'impulsions, des variations aléatoires d'amplitude, de fréquence ou de largeur d'impulsions, des textes et une sélection de modes d'interaction d'un utilisateur.

8. Procédé selon la revendication 6, **caractérisé en ce que**, dans au moins une fenêtre d'adaptation de paramètres (60, 70, 80, 90), au moins une courbe de données de variation de paramètres constituée d'un certain nombre de points pour au moins un canal de stimulation est dessinée dans un cadre sélectionné de la fenêtre d'adaptation correspondante, dans laquelle les points de la courbe dessinée peuvent être déplacés pour modifier sa forme, la ou les courbes des paramètres définissant des rampes de largeur d'impulsions et/ou des rampes d'amplitude d'impulsions pour des interactions d'un utilisateur.

9. Procédé selon l'une des revendications 1 et 2, **caractérisé en ce que** la ou les séquences de données de stimulation codées composées et traduites dans la station d'ordinateur (1) sont enregistrées sur le support amovible de mémorisation, qui est une carte à puce (8), placé dans le dispositif de lecture et d'écriture (18) d'un appareil portable de stimulation électrique fonctionnelle (2).

10. Procédé selon la revendication 9, **caractérisé en ce que** la ou les séquences de données de stimulation codées sont transférées par voie sérielle dans un câble (5) reliant la station d'ordinateur (1) au dispositif de lecture et d'écriture (18) de l'appareil portable de stimulation électrique (2).

11. Procédé selon la revendication 9, **caractérisé en ce que** la ou les séquences de données de stimulation codées sont transférées de la station d'ordinateur (1) au dispositif de lecture et d'écriture de l'appareil portable de stimulation électrique par des moyens d'émission et de réception de signaux sans fil (9, 9a, 9b).

12. Procédé selon la revendication 9 dans lequel plusieurs appareils portables de stimulation électrique fonctionnelle (2, 2'), qui comprennent chacun un dispositif de lecture et d'écriture (18) recevant une carte à puce (8, 8') respective et plusieurs canaux de stimulation électrique pour le branchement d'électrodes de stimulation, sont connectés les uns aux autres, **caractérisé en ce que** des séquences de données de stimulation sont composées dans la fenêtre principale (40) de l'interface utilisateur graphique pour tous les canaux de stimulation des appareils portables de stimulation électrique fonctionnelle, **en ce que** les séquences de données de stimulation sont traduites dans des séquences de données de stimulation codées, dont chaque séquence comprend les numéros d'identification spécifique des blocs unitaires de fonctions de stimulation configurés, les séquences codées étant placées les unes après les autres dans un ordre chronologique fonction du numéro du canal de stimulation et de l'appareil portable de stimulation correspondant, **en ce que** les séquences codées sont transférées dans les dispositifs de lecture et d'écriture des appareils portables de stimulation respectifs, et **en ce que** les séquences codées sont enregistrées sur chaque carte à puce (8, 8') introduite dans le dispositif de lecture et d'écriture (18) de l'appareil respectif.

13. Procédé selon la revendication 12, **caractérisé en ce que**, lors de la composition des jeux de séquences de données de stimulation à enregistrer sur les cartes à puce (8, 8'), un bouton de priorité graphique (44) est sélectionné ou déplacé dans une position maître ou une position esclave pour chaque jeu de séquences composées afin de définir le rôle de priorité de chaque carte à puce à l'enregistrement des séquences de données de stimulation codées, le bouton étant placé sur la position maître pour une des cartes à puce (8, 8') pour qu'un des appareils portables de stimulation (2, 2') puisse jouer le rôle de maître en synchronisant les autres appareils qui jouent le rôle d'esclave lors de la génération des impulsions électriques, basées sur les séquences codées des cartes à puce, aux électrodes de stimulation.

14. Procédé selon l'une des revendications 1, 2 et 12, **caractérisé en ce que**, lors de la composition des séquences de données de stimulation, des boutons graphiques (44) représentés dans la fenêtre principale (40) sont sélectionnés ou déplacés chacun entre deux positions pour définir la forme des impulsions électriques et le mode de programmation des séquences de données de stimulation, tel que le mode de programmation basé sur le temps ou le mode de programmation basé sur les impulsions.

15. Procédé selon l'une des revendications 1 et 2, **caractérisé en ce que** plusieurs supports de mémorisation (8, 8') sont introduits successivement dans le dispositif de lecture et d'écriture (18) pour que des séquences de données de stimulation codées, qui ont été composées successivement dans la station d'ordinateur (1), soient enregistrées sur chacun d'entre eux, et **en ce que** lors de la composition de chaque jeu de séquences de données de stimulation à enregistrer sur chaque support de mémorisation (8, 8'), un bouton de priorité graphique (44) représenté dans la fenêtre principale (40) est sélectionné ou déplacé dans une position maître ou une position esclave pour définir le rôle de priorité du support de mémorisation correspondant, le bouton étant placé dans la position maître pour un des supports de mémorisation pour que lesdits supports puissent être introduits dans plusieurs appareils portables de stimulation électrique fonctionnelle (2, 2') connectés les uns aux autres, et qu'un des appareils puisse jouer le rôle de maître en synchronisant les autres appareils qui jouent le rôle d'esclave lors de la génération des impulsions électriques, basées sur les séquences codées des supports de mémorisation, aux électrodes de stimulation.

16. Procédé selon la revendication 1, **caractérisé en ce que** des valeurs limites supérieures ou des valeurs par défaut d'amplitude, de fréquence ou de largeur d'impulsions sont introduites par des moyens d'introduction de données (3a, 3b) de la station d'ordinateur (1) en des positions (45) définies dans la fenêtre principale (40) pour chaque séquence de données de stimulation de chaque canal de stimulation.

17. Procédé selon la revendication 1, **caractérisé en ce que** des séquences de données de stimulation sont composées sur la station d'ordinateur (1) pour plusieurs canaux de stimulation d'un appareil portable de stimulation électrique fonctionnelle (2), lesdites séquences comprenant au moins un bloc unitaire de fonctions relatif à la synchronisation entre les canaux de stimulation.

18. Procédé selon la revendication 1, **caractérisé en ce que**, dans une phase préliminaire, le support de mémorisation (8), qui comprend des séquences de données de stimulation codées préalablement mémorisées, est placé dans le dispositif de lecture et d'écriture (18) en communication avec la station d'ordinateur (1), **en ce qu'**une commande de lecture du support de mémorisation est transmise de la station vers la dispositif de lecture et d'écriture pour visualiser sur la fenêtre principale (40) de l'interface utilisateur graphique les séquences de données de stimulation, ainsi que les données de paramètres sur au moins une fenêtre d'adaptation (60, 70, 80, 90) de manière à pouvoir les vérifier et/ou les modifier, et **en ce qu'**une mémorisation dans la station d'ordinateur des données de stimulation du support de mémorisation est réalisée.

19. Installation de programmation de données de stimulation électrique, notamment pour la mise en oeuvre du procédé selon l'une des revendications précédentes, l'installation comprenant une station d'ordinateur (1), qui comprend une unité centrale de traitement (10) à programme de gestion de données de stimulation électrique et une interface utilisateur graphique (4), au moins un dispositif de lecture et d'écriture (18) en communication avec la station d'ordinateur (1), et au moins un support de mémorisation (8) placé dans le dispositif pour mettre en mémoire des données personnelles de stimulation électrique, **caractérisée en ce que** des moyens de mémorisation (11) de l'unité centrale comprennent une bibliothèque de blocs unitaires de fonctions de stimulation (11b) représentés chacun par une icône (51) spécifique dans une fenêtre de bibliothèque (50) dans l'interface utilisateur graphique, des moyens d'introduction de données (3a, 3b) de la station d'ordinateur (1) permettant de tirer lesdits blocs de la fenêtre de bibliothèque et de les introduire dans une fenêtre principale (40) de l'interface graphique pour la composition d'au moins une séquence de données de stimulation électrique d'au moins un canal de stimulation d'un appareil de stimulation électrique neuromusculaire ou fonctionnelle (2), et **en ce que** les moyens d'introduction de données permettent également de configurer certains blocs unitaires de la séquence et/ou d'adapter des paramètres de stimulation visualisés dans au moins une fenêtre d'adaptation de paramètres (60, 70, 80, 90) sur l'interface utilisateur graphique pour l'exécution des blocs unitaires de fonctions de stimulation placés dans la séquence de données de stimulation.

20. Installation selon la revendication 19, **caractérisée en ce que** le dispositif de lecture et d'écriture (18) fait partie d'un appareil de stimulation électrique neuromusculaire ou fonctionnelle (2).
